# FASCICULE DE BREVET EUROPEEN

(11) **EP 2 731 943 B1**
(45) Date de publication et mention de la délivrance du brevet: **18.01.2017**
(21) Numéro de dépôt: 12743513.9
(22) Date de dépôt: 12.07.2012
(51) Int. Cl.: C07D 401/12, C07D 209/60, C07D 213/74, C07D 401/14

(54) **NOUVEAUX AGENTS D'IMAGERIE, LEUR PROCEDE DE PREPARATION ET LES COMPOSITIONS PHARMACEUTIQUES QUI LES CONTIENNENT**
NEUARTIGE KONTRASTMITTEL, HERSTELLUNGSVERFAHREN DAFÜR UND PHARMAZEUTISCHE ZUSAMMENSETZUNGEN DAMIT
NOVEL IMAGING AGENTS, PROCESS FOR THE PREPARATION THEREOF AND PHARMACEUTICAL COMPOSITIONS CONTAINING SAME

(30) Priorité: 13.07.2011 FR 1102213
(43) Date de publication de la demande: 21.05.2014
(73) Titulaire: Centre National de la Recherche Scientifique (C.N.R.S.), 75794 Paris Cedex 16 (FR)
(72) Inventeur: LEPAPE, Alain, F-37130 Lignieres de Touraine (FR); LERONDEL, Stéphanie, F-45000 Orleans (FR); REVEILLON, Guillaume, F-91400 Orsay (FR); DELBOS, Jean-Marie, F-41200 Romorantin-Lanthenay (FR); DEMUYNCK, Luc, F-45000 Orleans (FR); GRANDON, Hubert, F-45000 Orleans (FR); LEFOULON, François, F-45000 Orleans (FR); TUCKER, Gordon, F-75017 Paris (FR)
(74) Mandataire: Regimbeau
(86) Numéro de dépôt international: PCT/FR2012/051667
(87) Numéro de publication internationale: WO 2013/007959

(56) Documents cités:
- US-B1- 6 558 649

## Description

La présente invention concerne de nouveaux agents d'imagerie, leur procédé de préparation, les compositions pharmaceutiques qui les contiennent et leur utilisation à des fins diagnostiques.

Les approches actuelles de diagnostic moléculaire d'une maladie donnée requièrent généralement des échantillons sanguins et tissulaires, de la chirurgie ou encore, dans le cas des expérimentations animales, des sacrifices. Apparue récemment, l'imagerie directe de cellules vivantes est devenue un outil fondamental pour étudier en temps réel des processus biologiques de façon non-invasive directement au sein d'un organisme vivant. Par exemple, l'imagerie optique en fluorescence permet de fournir des données fonctionnelles en temps réel dans les modèles tumoraux *in vivo* (Pagani-Gioanni et al, STAL 2009, 36, 41-47).

L'utilisation de sondes moléculaires ciblées pour l'imagerie *in vivo* implique l'utilisation d'un agent qui cible sélectivement un gène, une protéine, un récepteur ou une fonction cellulaire, lesquels sont les cibles spécifiques d'un stade particulier de l'évolution d'une pathologie. Cette technique d'imagerie permet la mesure et/ou la visualisation des molécules-cibles et des voies moléculaires *in vivo* tout en donnant des informations sur les processus physiopathologiques au niveau moléculaire.

Les intégrines sont des récepteurs d'adhésion cellulaire transmembranaires situés à la surface de la cellule qui se lient aux molécules de la matrice extracellulaire. Une fois le récepteur activé, une cascade de signaux intracellulaires est déclenchée provoquant divers phénomènes biologiques tels que la croissance cellulaire, la survie, la différentiation et l'apoptose.

Structurellement, les intégrines sont des protéines hétérodimériques constituées d'une sous-unité α et d'une sous-unité β. A ce jour, il existe 18 sous-unités α et 8 sous-unités β différentes, et leur combinaison détermine la spécificité de l'intégrine au ligand. Parmi les différentes combinaisons possibles, on distingue les intégrines αᵥ, α₅β₁ et α_{IIb}β₃ qui reconnaissent la séquence arginine-glycine-acide aspartique (RGD) chez les protéines de la matrice extracellulaire. Les récepteurs à vitronectine αᵥβ₃ et αᵥβ₅, ainsi que le récepteur à fibronectine α₅β₁ régulent la migration et l'adhésion des cellules cancéreuses *via* la séquence RGD (Albelda et al, Cancer Res. 1990, 50, 6757-64 ; Gladson et al, J. Clin. Invest. 1991, 88, 1924-32; Lafrenie et al, Eur. J. Cancer 1994, 30, 2151-58). En particulier, les intégrines αᵥβ₃ et αᵥβ₅ sont exprimées dans de nombreuses cellules tumorales telles que le glioblastome (Bello et al, Neurosurgery 2001, 49, 380-9), le mélanome (Albelda et al, Cancer Res. 1990, 50, 6757-64) et les carcinomes du sein (Felding-Habermann et al, PNAS 2001, 98, 1853-8), du poumon (Wayner et al, J. Cell Biol. 1991, 113, 919-29) et des ovaires (Landen et al, Neoplasia 2008, 10, 1259-67).

L'angiogénèse est un processus physiologique normal décrivant la croissance de nouveaux vaisseaux sanguins (néovascularisation) à partir de vaisseaux préexistants. Elle intervient lors du développement embryonnaire, la réparation tissulaire mais aussi dans la croissance des tumeurs malignes et le développement des métastases. En particulier, les intégrines αᵥβ₃ et αᵥβ₅ sont fortement exprimées dans les cellules endothéliales activées lors de la néovascularisation tumorale mais faiblement présentes dans les cellules endothéliales quiescentes et dans la plupart des tissus sains (Brooks et al, Science 1994, 264, 569-71). Enfin, des observations cliniques ont montré une corrélation directe entre les taux d'expression des intégrines et la progression tumorale (Gasparini et al, Clin. Cancer. Res. 1998, 4, 2625-34; Bachmann et al, BMC Cancer 2008, 8, 362). Par conséquent, les intégrines αᵥβ₃ et αᵥβ₅ représentent une cible attractive dans la détection de la croissance tumorale et métastatique.

Il apparaît de ce qui précède que les techniques d'imagerie capables de localiser spécifiquement la cible moléculaire pourraient améliorer significativement la capacité du praticien à diagnostiquer et à déterminer un traitement efficace suivant le stade d'évolution de la maladie (depuis le développement précoce d'une tumeur primaire jusqu'à l'apparition de métastases). Elles permettraient d'évaluer les effets anti-tumoraux d'une drogue dans des études d'efficacité ou de guider par imagerie peropératoire le chirurgien au cours d'une résection tumorale.

Dans la littérature, des agents utilisés pour l'imagerie optique en fluorescence ciblant spécifiquement les intégrines ont été développés (WO 2009/114776 ; Kossodo et al, Mol. Imaging Biol. 2010, 12, 488-99 ; Ye et al, Theranostics 2011, 1, 102-26). D'autres agents d'imagerie comportant un radioisotope ont été aussi décrits (US 6,818,201).

Malgré de réels progrès dans l'imagerie non-invasive, des méthodes et des agents d'imagerie plus sensibles et plus spécifiques sont encore nécessaires. En particulier, il est intéressant de disposer de sondes capables de détecter un plus large spectre de type de tumeurs, d'améliorer le contraste entre la zone tumorale et les sites non-spécifiques et de minimiser l'accumulation dans les tissus sains.

Les composés de l'invention, outre le fait qu'ils soient nouveaux, concernent des agents d'imagerie aux caractéristiques pharmacologiques intéressantes leur conférant des propriétés qui les rendent utiles dans l'imagerie diagnostique de cancers difficiles à dépister et dans des situations où l'environnement tumoral complique une discrimination précise entre tissu sain et structures tumorales. Il s'agit en particulier des foyers profonds thoraciques et abdominaux et dans les territoires présentant un bruit de fond tissulaire ou vasculaire en imagerie peropératoire ou en imagerie fibroscopique.

Plus spécifiquement, la présente invention concerne les composés de formule (I) : 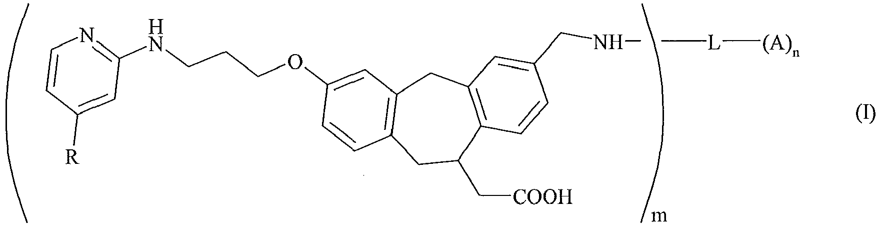 dans laquelle :
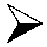 R représente un groupement alkyle (C₁-C₆) linéaire ou ramifié ;
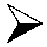 L représente un groupement espaceur qui est une chaîne hydrocarbonée, linéaire ou ramifiée, saturée ou insaturée, en C₁-C₂₀, étant entendu :
   - qu'un ou plusieurs groupements méthylène peuvent également être remplacés par un atome d'oxygène : un groupement -NH- ; un groupement -CO- ; un groupement hydroxy ; un groupement phényle : ou un groupement pyridine ; et
   - que ladite chaîne hydrocarbonée forme une liaison peptidique -NH-CO- avec l'aminé primaire du ligand de ciblage ;
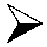 A représente un agent diagnostique correspondant à une entité chimique détectable par médecine nucléaire, par fluorescence, par radioactivité ou par détection optique ;
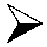 m et n sont chacun indépendamment égaux à 1 ou 2 ;
leurs énantiomères, leurs diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, acétique, trifluoroacétique, lactique, malonique, succinique, glutamique, fumarique, maléique, phosphorique, citrique, oxalique, méthane sulfonique, benzène sulfonique, para-toluènesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par ligand de ciblage, on entend le composé de formule (II) suivant : 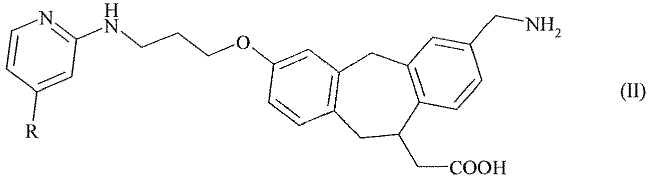 dans laquelle R est tel que défini dans la formule (I).

Plus particulièrement, le ligand de ciblage est l'acide (7-(aminométhyl)-3-{3-[(4-méthyl-2-pyridinyl)amino]propoxy}-10,11-dihydro-5*H*-dibenzo[*a*,*d*]cyclohepten-10-yl)acétique.

Dans les composés de formule (I), R représente préférentiellement un groupement méthyle.

Par groupement espaceur, on entend un groupement moléculaire fonctionnalisé utilisé pour lier chimiquement de manière covalente un ou plusieurs ligands de ciblage à un ou plusieurs agents diagnostiques identiques ou différents.

Le groupement espaceur L de la présente invention représente une chaîne hydrocarbonée, linéaire ou ramifiée, saturée ou insaturée, en C₁-C₂₀, étant entendu :
- qu'un ou plusieurs groupements méthylène peuvent également être remplacés par un atome d'oxygène ; un groupement -NH- ; un groupement -CO- ; un groupement hydroxy ; un groupement phényl ; ou un groupement pyridyl et
- que ladite chaîne hydrocarbonée forme une liaison peptidique -NH-CO- avec l'amine primaire du ligand de ciblage.

Selon un mode de réalisation particulier de l'invention, L peut représenter un dérivé d'un acide aminé naturel ou non-naturel, non-cyclique, étant entendu que la fonction acide dudit acide aminé située sur le carbone α et/ou sur la chaîne latérale lorsqu'elle existe, forme une liaison peptidique avec l'amine primaire du ligand de ciblage.

Préférentiellement, le groupement espaceur L représente un groupement -CO- ; un groupement (C₁-C₆) alkylcarbonyle ; un groupement phénylcarbonyle ; ou un groupement pyridylcarbonyle.

Selon un mode de réalisation préféré, L représente le groupement *n*-pentylcarbonyle.

Selon un autre mode de réalisation avantageux, le groupement espaceur L représente un dérivé de l'acide 6-aminocaproïque, et plus particulièrement, le groupement -CO-(CH₂)₅-NH- ; un dérivé de l'acide glutamique, et plus particulièrement, le groupement 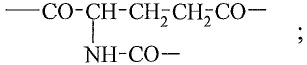 ou un dérivé de la lysine, et plus, particulièrement le groupement

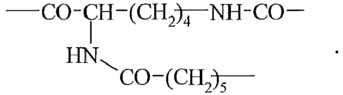

Par agent diagnostique, on entend une entité chimique détectable par médecine nucléaire (par exemple, scintigraphie), par fluorescence, par radioactivité ou par détection optique, permettant la visualisation d'une structure anatomique (par exemple, un organe) ou pathologique (par exemple, une tumeur) difficilement distinguable des tissus voisins. Parmi les entités chimiques disponibles envisagées comme agent diagnostique, on choisira préférentiellement un marqueur radioactif, un ion métallique paramagnétique, un fluorophore ou une nanoparticule (communément appelée « quantum dot »).

Par marqueur radioactif, on entend tout atome à noyau instable et radioactif (radioisotope) des métaux tels que le cuivre, l'indium ou le technétium, ou encore les radioisotopes des atomes d'halogènes, tels que l'iode ou le fluor. Parmi les radioisotopes des éléments ci-dessus, on peut citer, par exemple, ⁶⁴Cu, ⁶⁷Cu, ¹¹¹In, ^{99m}Tc, ¹²³I, ¹²⁵I, ¹⁸F, etc...

Par ion métallique paramagnétique, on entend un ion d'un métal de transition qui ne possède pas d'aimantation spontanée mais qui, sous l'effet d'un champ magnétique extérieur, acquiert une aimantation dirigée dans le même sens que ce champ d'excitation. Parmi ces ions métalliques, on peut citer, par exemple, Gd(III), Mn(II), Fe(III), etc...

Les marqueurs métalliques radioactifs et les ions métalliques paramagnétiques sont liés sur l'agent d'imagerie par chélation à l'aide d'un chélateur.

Par chélateur, on entend désigner une entité liée de manière covalente sur l'agent d'imagerie au niveau du groupement espaceur et susceptible de fixer par chélation un radioisotope ou un ion métallique paramagnétique. Parmi les chélateurs capables de former des complexes stables avec les cations métalliques, on peut citer à titre non limitatif les diamine-dithiols, les triamide-monothiols, les diamine-dioximes, les hydrazines, les acides polyaminocarboxyliques (DTPA, DOTA ...), etc ...

Par fluorophore, on entend toute substance chimique capable d'émettre de la lumière de fluorescence après excitation. Parmi les fluorophores commercialement disponibles utiles dans la conception des agents d'imagerie de la présente invention, on peut citer à titre non limitatif la fluorescéine; les cyanines Cy™3, Cy™5, Cy™5.5 et Cy™7 (GE Healthcare) ; Alexa Fluor® 660, Alexa Flour® 680, Alexa Fluor® 750 et Alexa Fluor® 790 (Invitrogen) ; VivoTag®680, VivoTag-S®680 et VivoTag-S®750 (VisEn Médical); Dy™677, Dy™682, Dy™752 et Dy™780 (Dyomics); DyLight® 547 et DyLight® 647 (Pierce) ; etc...

De manière avantageuse, les composés de formule (I) sont les composés pour lesquels A représente un fluorophore. Le groupement A représente préférentiellement la fluorescéine, l'Alexa Fluor® 750, le Cy™3, le Cy™5.5 ou le Cy™7. Plus particulièrement, le fluorophore préféré est le Cy™5.5.

Des composés préférés de l'invention sont ceux pour lesquels A représente un radioisotope de l'iode adapté à la modalité d'imagerie envisagée : ¹²³I pour la scintigraphie, ¹²⁴I pour la tomographie par émission de positon, voire ¹²⁵I pour l'imagerie du petit animal.

Une autre possibilité préférée pour les composés de formule (I) consiste en ce que A représente le groupement hydrazine associé au radioisotope ^{99m}Tc.

Selon un premier mode réalisation préféré, m et n sont égaux à 1.

Un mode de réalisation avantageux consiste en ce que m est égal à 1 et n est égal à 2. Dans ce dernier mode de réalisation, les agents diagnostiques sont préférentiellement différents. En particulier, on choisira un premier agent diagnostique représenté par un fluorophore et un deuxième agent représenté par un radioisotope associé avec un chélateur.

Les composés préférés de l'invention sont les composés représentés par les formules suivantes :

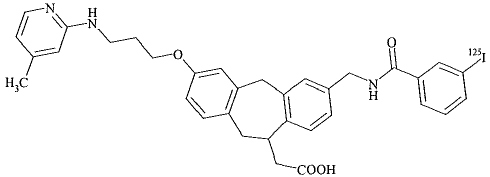

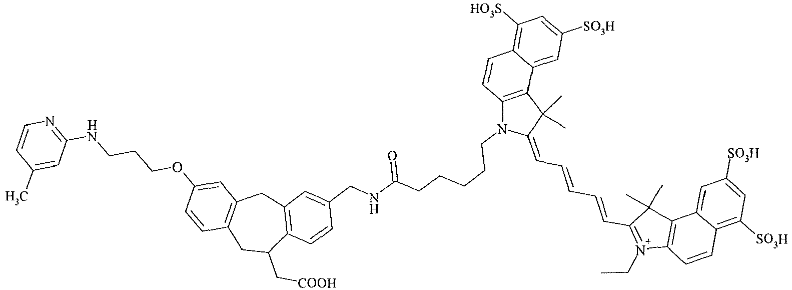

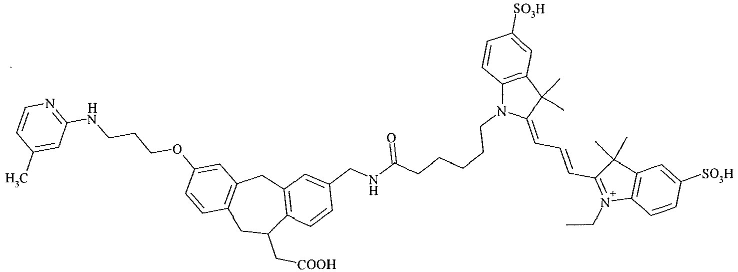

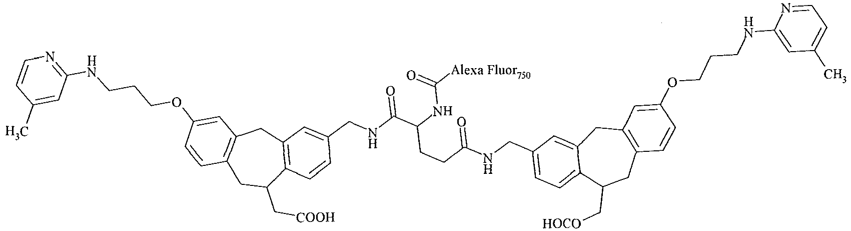

Les sels d'addition à un acide ou une base pharmaceutiquement acceptable des composés préférés de l'invention font partie intégrante de l'invention.

L'invention s'étend également au procédé de préparation des composés de formule (I), caractérisé en ce que l'on utilise comme produit de départ un composé de formule (II) : 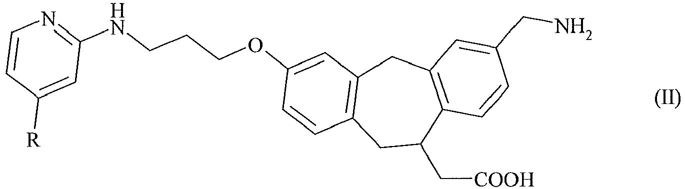 dans laquelle R est tel que défini dans la formule (I),
qui est mis en réaction avec un composé de formule (III) : 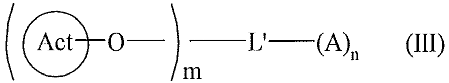 dans laquelle :
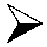 A, m et n sont tels que définis dans la formule (I) ;
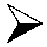 Act représente un activateur de couplage peptique, tel que le *N*-hydroxysuccinimide (NHS), l'éthyl(diméthylaminopropyl)carbodiimide (EDC) ou l'hydroxybenzothiazole (HOBt) ;
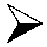 L' représente une chaîne hydrocarbonée, linéaire ou ramifiée, saturée ou insaturée, en C₁-C₂₀, étant entendu :
   - qu'un ou plusieurs groupements méthylène peuvent également être remplacés par un atome d'oxygène ; un groupement -NH- ; un groupement -CO- ; un groupement hydroxy ; un groupement phényle ; ou un groupement pyridine ; et
   - que la chaîne hydrocarbonée forme une liaison ester avec l'activateur Act ;
   pour conduire au composé de formule (I),
   composé de formule (I) qui peut être ensuite purifié selon une technique classique de séparation.

Les composés de formule (III) sont commerciaux ou aisément accessibles à l'homme du métier par des réactions de chimie classiques ou décrites dans la littérature.

Le composé de formule (II) est nouveau et fait également partie de l'invention à titre de ligand de ciblage des récepteurs à intégrines utiles dans la préparation des composés de formule (I).

Les composés de formule (I) selon l'invention sont utiles en tant que nouvel agent d'imagerie pour l'évaluation de la fonction des organes ou de zones tumorales.

Fait également partie du domaine de la présente invention, l'utilisation du composé de formule (I) pour évaluer les effets de l'administration d'un médicament chez l'animal ou chez l'humain atteint d'une pathologie au cours de laquelle une néovascularisation intervient.

L'ajustement des quantités efficaces adéquates du composé de formule (I) pour la mise en oeuvre dans une utilisation selon l'invention dépend du phénomène ou de l'entité à détecter/quantifier. *In vivo*, les quantités efficaces peuvent, également, être ajustées selon la taille, le poids de l'individu chez qui l'utilisation selon l'invention est mise en oeuvre, ainsi que selon l'organe, le tissu ou les cellules ciblées. Les ajustements peuvent être réalisés par toutes méthodes usuellement pratiquées par l'homme de l'art.

Au sens de la présente invention, on entend par « quantité efficace », la quantité nécessaire et suffisante pour obtenir l'effet désiré, à savoir la détection et/ou la quantification d'une entité biologique ou d'un phénomène chimique, physique ou biologique.

Dans le cadre de leur mise en oeuvre *in vivo* ou *in vitro,* le composé de formule (I) peut être mis en forme afin d'être adapté à une administration par voie orale, ou parentérale, notamment intraveineuse, intra-artérielle, intracardiaque, intracérébro-ventriculaire, intrapéritonéale, intratumorale, ou pour une administration par voie pulmonaire, nasale, ophtalmique et éventuellement rectale, vaginale ou topique.

L'invention s'étend également aux compositions pharmaceutiques renfermant comme principe actif au moins un composé de formule (I) seul ou en combinaison avec un ou plusieurs excipients ou véhicules inertes non toxiques. Le composé de formule (I) peut être ainsi mis en oeuvre dans une composition pharmaceutique adaptée au procédé de détection à réaliser et à la voie d'administration choisie.

Le composé de formule (I) selon l'invention peut être administré à un être vivant, tel que par exemple un animal ou un être humain, puis la détection du marqueur peut être réalisée *in vivo* selon les moyens usuellement mis en oeuvre dans le domaine.

Selon un autre mode de réalisation, la présente invention se rapporte à un kit de préparation d'un agent d'imagerie comprenant au moins un composé de formule (I) dans lequel A représente, en particulier, un radioisotope associé à un chélateur. Dans ce cas, le kit inclut un ou plusieurs récipients scellés contenant une quantité prédéterminée d'un agent d'imagerie comportant ledit chélateur. Selon un certain mode de réalisation, le kit peut comporter un second récipient scellé contenant un marqueur radioactif.

Le kit peut aussi contenir des adjuvants pharmaceutiques conventionnels tels que des sels pharmaceutiquement acceptables pour ajuster la pression osmotique, des tampons, des agents de conservation, des diluants, des émulsifiants, des excipients etc...

Le kit peut notamment s'agir d'une trousse de marquage prête à l'emploi pour des équipes travaillant sur des modèles expérimentaux de pathologie déterminée.

Les exemples et les figures présentés ci-après illustrent l'invention et ne la limitent en aucune façon. Les structures des composés décrits ont été confirmées par les techniques spectroscopiques usuelles : RMN du proton (s = singulet ; ls = large singulet ; tls = très large singulet ; d = doublet ; ld = large doublet ; t = triplet ; lt = large triplet ; dd = doublet dédoublé ; q = quadruplet ; lq = large quadruplet ; qt = quintuplet ; lqt = large quintuplet ; m = multiplet ; 2s = 2 singulets ; 2d = 2 doublets) ; spectrométrie de masse par impact électronique (EI) ou par ionisation par électrospray (ESI).

### Légende des figures :

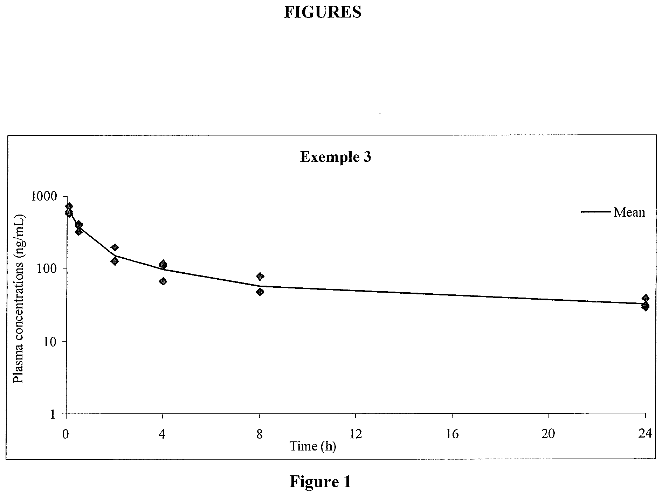

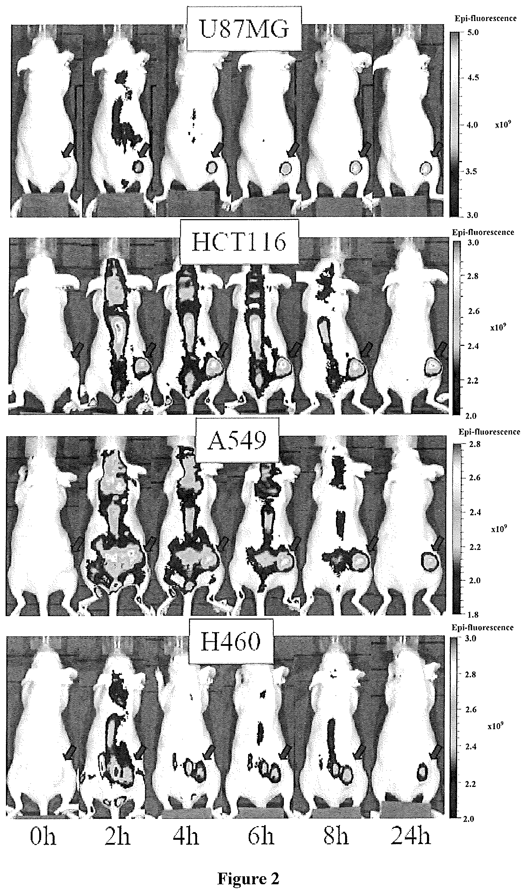

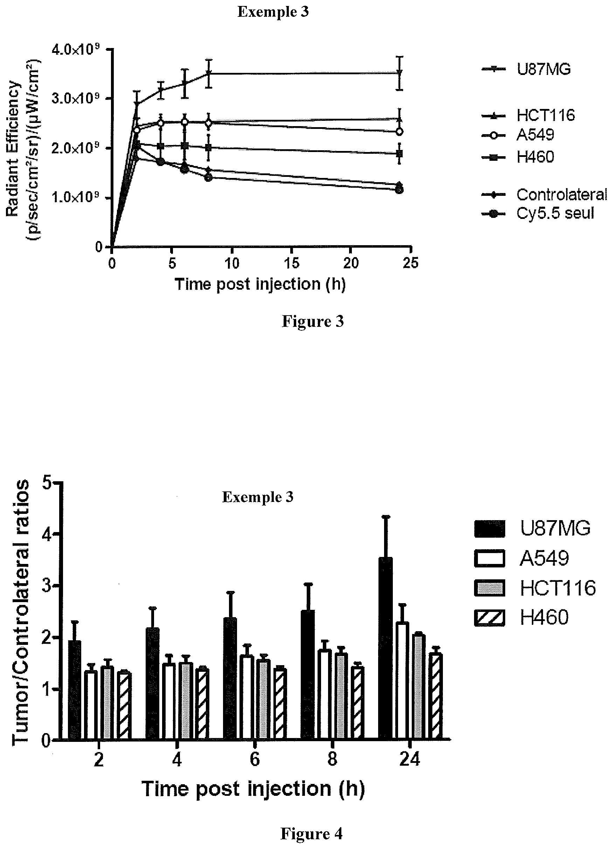

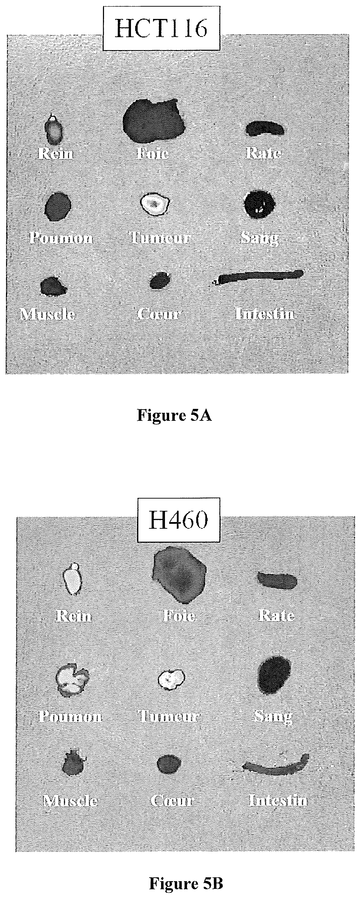

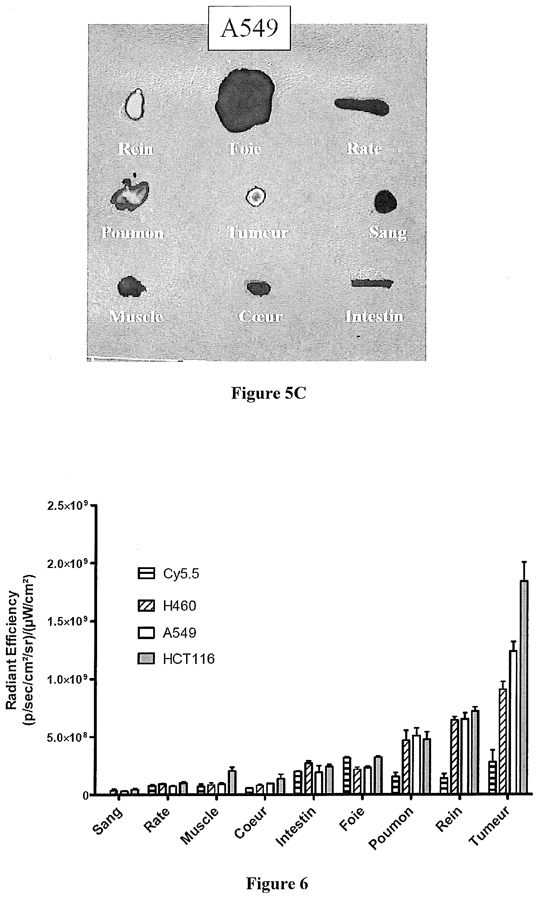

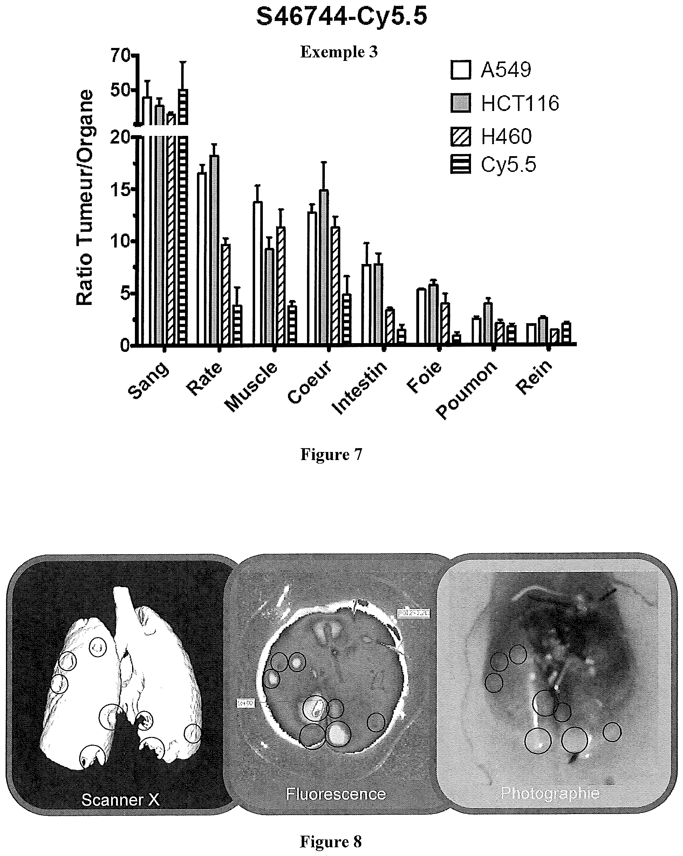

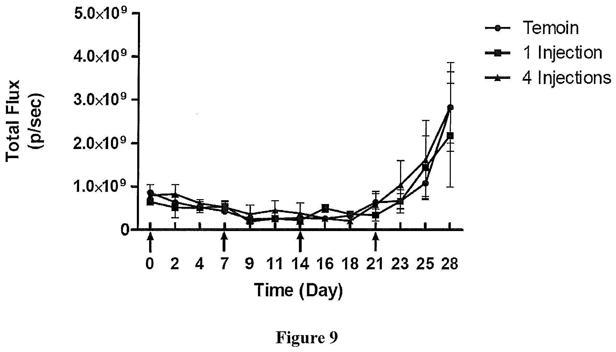

▪ Figure 1 : Cinétique de la concentration plasmatique moyenne (ng/mL) après injection intraveineuse du composé de l'Exemple 3 (2 nmol/animal) chez des souris Swiss nude.
▪ Figure 2 : Imagerie de fluorescence IR du composé de l'Exemple 3 à 2h, 4h, 6h, 8h, 24h post-injection dans des modèles de xénogreffes tumorales U87MG (glioblastome), HCT116 (carcinome colorectal), A549 (adénocarcinome pulmonaire) et H460 (carcinome pulmonaire).
▪ Figure 3 : Cinétique *in vivo* de la fluorescence émise par le composé de l'Exemple 3 et par la Cyanine5.5 seule au sein des différents modèles de tumeurs et de la région controlatérale.
▪ Figure 4 : Rapport de fluorescence entre la tumeur et la zone controlatérale pour toutes les lignées cancéreuses utilisées.
▪ Figure 5 : Imagerie *ex vivo* de fluorescence IR à t₂₄ₕ du composé de l'Exemple 3 dans les différents organes, tissus et tumeurs (A : HCT116 ; B : H460 ; C : A549).
▪ Figure 6 : Quantification *ex vivo* à t₂₄ₕ de la biodistribution du composé de l'Exemple 3 dans les différents organes, tissus et tumeurs.
▪ Figure 7 : Evaluation du ratio Tumeur/Organe à t₂₄ₕ du composé de l'Exemple 3 dans les organes, tissus et tumeurs.
▪ Figure 8 : Colocalisation multimodale des lésions tumorales spontanées dans le modèle murin K-rasLA1.
▪ Figure 9 : Cinétique de la croissance tumorale par bioluminescence avec ou sans injection du composé de l'Exemple 3 chez des souris Swiss nude.

### Préparation 1 : Acide (7-(aminométhyl)-3-{3-[(4-méthyl-2-pyridinyl)amino]propoxy} -10,11-dihydro-5H-dibenzo[a,d]cyclohepten-10-yl)acétique

### Stade A : 3-(3-bromophényl)-1H-indén-5-yl méthyl éther

A une solution de 1-bromo-3-iodobenzène (339 mmol) dans 600 mL de THF anhydre à - 40 °C, est additionnée goutte à goutte une solution de *n*-dibutylmagnésium 1 M dans l'heptane (185 mmol). La 6-méthoxyindanone (154 mmol) est ensuite additionnée par petites portions à - 40 °C. L'agitation est poursuivie pendant 24 heures, en laissant la température remonter à l'ambiante. Après hydrolyse par 300 mL de HCl 1 N, la phase aqueuse est extraite 2 fois par l'acétate d'éthyle. Les phases organiques sont rassemblées, séchées sur sulfate de sodium, concentrées et concrétisées dans de l'éther isopropylique pour obtenir le produit attendu.
*Spectrométrie de masse (EI) : 300 [M*^{+•}*] ; 285 [M-CH₃^{•}]*⁺ *; 269 [M-OCH₃•]*⁺*.*

### Stade B : Acide [2-(3-bromobenzoyl)-4-méthoxyphényl]acétique

A une solution du produit du Stade A précédent (153 mmol) dans 250 mL d'acétone à 0 °C, est additionnée une solution de trioxyde de chrome 3,8 M dans l'acide sulfurique (459 mmol). Après agitation à 0 °C pendant 30 minutes, le milieu réactionnel est versé dans un mélange éther/glace. La phase aqueuse est extraite par de l'éther puis les phases organiques sont rassemblées, lavées à l'eau, séchées sur sulfate de sodium, concentrées et recristallisées dans l'acétate d'éthyle pour obtenir le composé attendu.
*Spectrométrie de masse (EI) : 350 [M*^{+•}*] ; 304 [M*^{+•}*-CO₂H] ; 225 [305-Br].*

### Stade C : Acide [2-(3-bromobenzyl)-4-méthoxyphényl]acétique

A une solution du composé du Stade B précédent (30 mmol) dans 50 mL d'acide trifluoroacétique refroidie dans un bain d'acétone/glace, est ajouté par petites fractions le cyanoborohydrure de sodium (90 mmol). L'agitation est poursuivie à température ambiante pendant 24 heures. Le milieu réactionnel est alors versé dans un mélange glace/acétate d'éthyle. Une solution de soude 1 N est ajoutée jusqu'à obtention d'un pH voisin de 5. La phase aqueuse est extraite par de l'acétate d'éthyle. Les phases organiques sont séchées sur sulfate de sodium, filtrées, concentrées et purifiées sur colonne de silice (éluant : dichlorométhane / méthanol 99 / 1) pour obtenir le composé attendu. *Spectrométrie de masse (EI) : 332 [M*^{+•}*].*
*Spectrométrie de masse (ESI*+*) : 333 [M*+*H]*⁺*; 355 [M*+*Na]*⁺*.*

### Stade D : 7-bromo-3-méthoxy-5,11-dihydro-10H-dibenzo[a,d]cyclohepten-10-one

A une solution du composé du Stade C précédent (32 mmol) dans le dichlorométhane et quelques gouttes de diméthylformamide, est ajoutée goutte à goutte une solution de chlorure d'oxalyle 2 M dans le dichlorométhane (97 mmol). Le milieu réactionnel est agité à température ambiante pendant 1 heure, concentré, séché puis repris dans le dichlorométhane à 0 °C. Du trichlorure d'aluminium (65 mmol) est ensuite ajouté par petites fractions. Après agitation à 0 °C pendant 30 minutes, le milieu réactionnel est versé dans un mélange glace/dichlorométhane. La phase aqueuse est extraite avec du dichlorométhane. Les phases organiques sont rassemblées, séchées sur sulfate de sodium, filtrées, concentrées et purifiées sur colonne de silice (éluant : dichlorométhane) pour obtenir le composé attendu.
*Spectrométrie de masse (EI) : 318 [M*^{+•}*] ; 237 [M*^{+•}*-Br].*

### Stade E : 7-méthoxy-11-oxo-10,11-dihydro-5H-dibenzo[a,d]cycloheptène-3-carbonitrile

A une solution du composé du Stade D précédent (8,5 mmol) dans 15 mL de diméthylformamide, sont additionnés le Zn(CN)₂ (8,5 mmol) et le Pd(PPh₃)₄ (0,85 mmol). Le milieu réactionnel est agité à 90 °C pendant 3 heures puis concentré et purifié sur colonne de silice (éluant : dichlorométhane / cyclohexane 70/30) pour obtenir le composé attendu.
*Spectrométrie de masse (EI) : 263 [M*^{+•}*] ; 232 [M-OCH₃^{•}]*⁺ *; 204 [232-CO] ; 177 [204-HCN].*

### Stade F : (7-cyano-3-méthoxy-5H-dibenzo[a,d]cyclohepten-10yl)acétate d'éthyle

Une solution d'hexaméthyldisilazide de lithium 1 N dans le THF (140 mL) est additionnée goutte à goutte à 13 mL d'acétate d'éthyle sous courant d'argon à - 78 °C. Après 15 minutes d'agitation, une solution du composé du Stade E précédent (22 mmol) dans 200 mL de THF est ajoutée goutte à goutte. L'agitation est poursuivie à - 78 °C pendant 45 minutes. Le milieu réactionnel est alors versé dans un mélange glace/éther. La phase aqueuse est extraite par de l'éther. Les phases organiques sont rassemblées, séchées sur sulfate de sodium, filtrées, concentrées puis mises en solution dans 50 mL d'acide trifluoroacétique. Le milieu est agité à température ambiante pendant 1,5 heures, puis est versé dans un mélange glace/éther/NaHCO₃. La phase aqueuse est extraite par de l'éther. Les phases organiques sont rassemblées, séchées sur sulfate de sodium, filtrées et concentrées pour obtenir le composé attendu.
*Spectrométrie de masse (ESI*+*) : 334 [M+H]⁺ ; 356* [*M*+*Na*]⁺.

### Stade G : (3-méthoxy-7-{[(trifluoroacétyl)amino]méthyl}-5H-dibenzo[a,d] cyclohepten-10-yl)acétate d'éthyle

Le composé du Stade F précédent (41 mmol) dans 1 L d'éthanol, 250 mL d'ammoniaque à 25 % et 1 g de Nickel de Raney sont mis sous pression d'hydrogène (40-50 psi) pendant 4 heures. Après filtration, le milieu est concentré et le résidu est dissous dans 30 mL de dichlorométhane, puis 30 mL d'eau sont ajoutés. La phase aqueuse est extraite par du dichlorométhane. Les phases organiques sont rassemblées, séchées sur sulfate de sodium, filtrées et concentrées. Le produit obtenu est traité par une solution d'anhydride trifluoroacétique (0,2 mol) et de triéthylamine (41 mmol) dans 40 mL de dichlorométhane. Le milieu est agité à température ambiante pendant 3 heures, concentré et repris par 200 mL de dichlorométhane. La phase organique est lavée par 200 mL d'eau, séchée sur sulfate de sodium, filtrée, concentrée et purifiée sur colonne de silice (éluant : cyclohexane / acétate d'éthyle 70/30) pour obtenir le composé attendu.
*Spectrométrie de masse (EI) : 433 [M*^{+•}*] ; 360 [M-C₂H₅CO₂^{•}]⁺ ; 346 [M-C₂H₅CO₂CH₂^{•}]*⁺.
*Spectrométrie de masse (ESI*+*) : 434 [M+H]⁺ ; 456 [M+Na]*⁺ *; 472 [M*+*K]*⁺.

### Stade H : (3-méthoxy-7-{[(trifluoroacétyl)amino]méthyl}-10,11-dihydro-5H-dibenzo[a,d]cyclohepten-10-yl)acétate d'éthyle

Le composé du Stade G précédent (25 mmol) et 1 g de Pd(OH)₂ dans 1 L d'éthanol sont mis sous pression d'hydrogène (60 psi) pendant 16 heures, puis la solution est filtrée et concentrée sous vide pour obtenir le composé attendu.
*Spectrométrie de masse (EI) : 435 [M*^{+•}*] ; 389 [M-HOC₂H₅]*^{+•} *; 347 [M-CH₃CO₂C₂H₅]*^{+•}*.*

### Stade I : (3-hydroxy-7-{[(trifluoroacétyl)amino]méthyl}-10,11-dihydro-5H-dibenzo[a,d]cyclohepten-10-yl)acétate d'éthyle

A une solution du composé du Stade H précédent (21,7 mmol) dans 100 mL de dichlorométhane à 0 °C, sont additionnés 67 mL de BBr₃. Après agitation à 0 °C pendant 1,75 heures, le milieu réactionnel est versé dans un mélange glace/NaHCO₃ 1 M/dichlorométhane. La phase aqueuse est extraite par du dichlorométhane. Les phases organiques sont rassemblées, séchées sur sulfate de sodium, filtrées, concentrées pour obtenir le composé attendu.
*Spectrométrie de masse (EI) : 421 [M*^{+•}*] ; 375 [M-HOC₂H₅]*⁺ *; 333 [M-CH₃CO₂C₂H₅]*^{+•}*.*

### Stade J: (3-{3-[(4-méthyl-1-oxydo-2-pyridinyl)amino]propoxy}-7-{[(trifluoroacétyl)amino]méthyl}-10,11-dihydro-5H-dibenzo[a,d] cyclohepten-10-yl)acétate d'éthyle

A une solution de 2[(3-hydroxypropyl)amino]-4-méthylpyridine-*N-*oxyde (42 mmol), de PPH₃ (42 mmol) et du composé du Stade I précédent (21 mmol) dans 430 mL de dichlorométhane à 0 °C, est additionnée goutte à goutte le azodicarboxylate de diisopropyle (42 mmol). Le mélange réactionnel est agité à température ambiante pendant 6 heures, concentré et purifié sur colonne de silice (éluant : dichlorométhane / méthanol 95 / 5) pour obtenir le composé attendu.
*Spectrométrie de masse (EI) : 585 [M*^{+•}*] ; 569 [M-O]*^{+•} *; 498 [M-CH₂CO₂C₂H₅^{•}]*⁺*.*
*Spectrométrie de masse (ESI*+*) : 586 [M*+*H]*⁺*.*

### Stade K : (3-{3-[(4-méthyl-2-pyridinyl)amino]propoxy}-7-{[(trifluoroacétyl) amino]méthyl}-10,11-dihydro-5H-dibenzo[a,d]cyclohepten-10-yl)acétate d'éthyle

Le composé du Stade J précédent (18 mmol) et Pd(OH)₂ (5,1 g) dans 130 mL d'isopropanol et 100 mL de cyclohexène sont chauffés à reflux pendant 12 heures. Le brut réactionnel est filtré, concentré et purifié sur colonne de silice (éluant : dichlorométhane / méthanol 97 / 3) pour obtenir le composé attendu.
*Spectrométrie de masse (ESI*+*) : 570 [M*+*H]*⁺ *; 1139 [2M*+*H]*⁺*.*

Les deux énantiomères sont séparés par chromatographie préparative sur colonne 250 x 50 mm Chiralpak IA 5 µm (éluant : dichlorométhane/heptane/diéthylamine 55/45/0,1).

### Stade L : Acide (7-(aminométhyl)-3-{3-[(4-méthyl-2-pyridinyl)amino]propoxy}-10,11-dihydro-5H-dibenzo[a,d]cyclohepten-10-yl)acétique

Une solution du composé du Stade K précédent (7 mmol) dans 30 mL d'une solution de soude 2 N et 75 mL d'éthanol est agitée à température ambiante pendant 12 heures. Le milieu réactionnel est extrait avec de l'éther diéthylique et la phase aqueuse est amenée à pH 8 avec une solution de HCl à 0 °C. Le précipité est filtré et séché pour obtenir le produit du titre.
*Point de fusion : 226 °C.*
*Pouvoir rotatoire : [α_{D}]^{20°}* = *- 32° (c = 5mg*/*ml, H₂O*/*CH₃CN 1:1).*
*Spectrométrie de masse (ESI*+*) : 429 [M-NH₃*+*H]*⁺ *; 446 [M*+*H]*⁺ ; *468 [M*+*Na]*⁺ ; *490 [M-H*+*2Na]*⁺*.*
*Analyse RMN ¹H (400 Mhz, DMSO, δ en ppm) : 1,94 (qt, 2H) ; 2,15 (s, 3H) ; 2,53-2,63 (dd, 2H) ; 2*,*84*/*3*,*21 (dd, 2H) ; 3,36 (q, 2H) ; 3, 68 (m, 1H*) *; 3,90-4,23 (d, 2H) ; 3,93 (s, 2H) ; 4,00 (t, 2H) ; 6*,*35 (s, 1H*) *; 6,36 (d, 1H) ; 6,69 (dd, 1H) ; 6*,*81 (d, 1H) ; 6,98 (d, 1H*) *; 7,21 (d, 1H) ; 7,28 (d, 1H*) *; 7,29 (s, 1H*) *; 7,81 (d, 1H*) *; 8,29 (ls, 3H) ; 12,24 (ls, 1H)*.

### Préparation 2 : Acide (7-{[(2-amino-5-{[(11-(carboxyméthyl)-7-{3-[(4-méthyl-2-pyridinyl)amino]propoxy}-10,11-dihydro-5H-dibenzo[a,d] cyclohepten-3-yl)méthyl]amino}-5-oxopentanoyl)amino]méthyl}-3-{3-[(4-méthyl-2-pyridinyl)amino]propoxy}-10,11-dihydro-5H-dibenzo[a,d]cyclohepten-10-yl)acétique

Une solution du composé de la Préparation 1 (0,387 mmol) et de 4-[(2,5-dioxo-1-pyrrolidinyl)oxy]-1-{[(2,5-dioxo-1-pyrrolidinyl)oxy]carbonyl}-4-oxobutylcarbamate de *tert-butyle* [ou Boc-Glu(OSu)-OSu] (0,193 mmol) dans 3 mL de diméthylformamide est agitée à température ambiante pendant 12 heures. Le brut réactionnel est purifié par chromatographe préparative puis le produit est repris avec 2 mL d'acide trifluoroacétique et agité à température ambiante pendant 4 heures pour donner le composé du titre.

### Conditions de la chromatographie préparative :

- colonne Symmetry C18 7 µm ;
- éluant A : eau / 0,1 % TFA ;
- éluant B : acétonitrile / 0,1 % TFA ;
- Gradient de 25 à 90 % de B en 30 minutes à 70 mL/min ;
- Temps de rétention = 23,77 minutes.

*Analyse RMN ¹H(400 MHz, DMSO, δ en ppm) : 1,98 (m, 6H) ; 2,25 (s, 6H) ; 2,27 (m, 2H) ; 2*,*45 (dd, 2H) ; 2,57 (dd, 2H) ; 2*,*81 (m, 2H) ; 3,19 (m, 2H) ; 3*,*40* (*m*, *4H) ; 3,63 (m, 2H) ; 3*,*81 (m, 1H) ; 3,85 (dd, 2H) ; 4,00 (t, 4H) ; 4*,*20 (m, 5H) ; 4,31 (dd, 1H) ; 6,62 (ld, 2H) ; 6,69 (m, 4H) ; 6,79 (d, 2H) ; 6,97 (dd, 2H) ; 7,00-7,15 (m, 6H) ; 7*,*80 (d, 2H) ; 8,17 (tls, 3H) ; 8*,*42 (t, 1H) ; 8,84 (lt, 1H) ; 12*,*25 (ls, 2H).*

### EXEMPLE 1 : Conjugué fluorescéine du composé de la Préparation 1

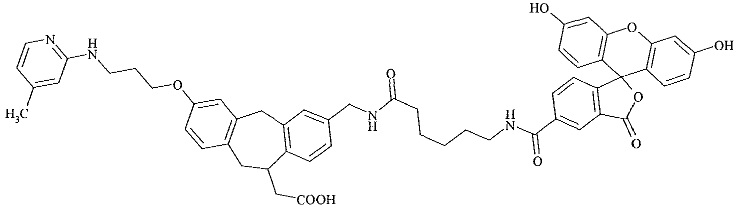

Une solution de composé de la Préparation 1 (35 µmol) et d'ester *N*-hydroxysuccinimide d'acide fluorescein-5(6)-carboxamidocaproïque (43,5 µmol) dans 5 mL de THF et 2 mL de diméthylformamide est agitée à température ambiante pendant 12 heures. Le brut réactionnel est purifié par chromatographie préparative pour obtenir le composé du titre.

### Conditions de la chromatographie préparative :

- colonne Chromasil C18 5 µm ;
- éluant A : eau / 0,1 % TFA ;
- éluant B : acétonitrile / 0,1 % TFA ;
- Gradient de 20 à 100 % de B en 30 minutes à 1 mL/min ;
- Temps de rétention = 15,14 minutes.

*Analyse RMN ¹H (400 Mhz, DMSO, δ en ppm) : 1,24-1,34 (qt, 2H) ; 1,49 (m, 2H) ; 1,58 (qt, 2H) ; 2*,*01 (lqt, 2H) ; 2,09-2,15 (t, 2H) ; 2,30 (s, 3H) ; 2,45 (m, 1H) ; 2*,*58* (*m*, *1H) ; 2*,*83 (dd, 1H) ; 3,19 (m, 1H) ; 3*,*19*-*3*,*31 (q, 2H) ; 3*,*45 (lq, 2H) ; 3*,*62 (m, 1H) ; 3*,*86 (dd, 1H) ; 4*,*03 (m, 2H) ; 4,16 (m, 3H) ; 6*,*57 (m, 4H) ; 6*,*70 (m, 4H) ; 6*,*81 (m, 2H) ; 6,95-7,15 (m, 4H) ; 7*,*36*-*8*,*07 (d, 1H) ; 7*,*67*-*8*,*46 (s, 1H) ; 7*,*79 (d, 1H) ; 8,15-8,25 (m, 2H) ; 8*,*51 (ls, 1H) ; 8*,*65*-*8*,*79 (lt, 1H) ; 10,14 (ls, 2H) ; 12*,*20 (ls, 1H) ; 12*,*95 (ls, 1H).*

### EXEMPLE 2 : Conjugué Cy™ 3 du composé de la Préparation 1

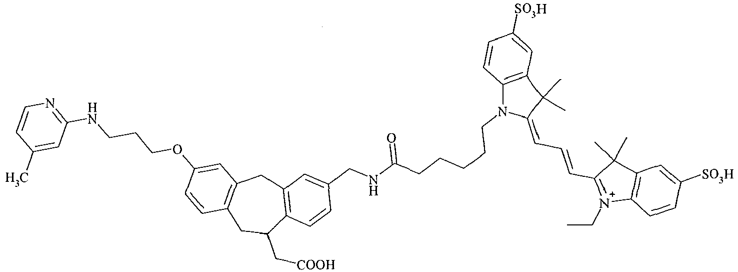

Une solution de composé de la Préparation 1 (13,5 µmol), de Cyanin3 MonoNHS ester (référence PA13102 GE Healthcare) (13 µmol) et de 100 µL de triéthylamine dans 2 mL de DMSO est agitée à température ambiante pendant 12 heures. Le brut réactionnel est purifié par chromatographie préparative pour donner le composé attendu.

### Conditions de la chromatographie préparative :

- colonne XTerra MS C18 2,5 m;
- éluant A : eau / acétonitrile /acide méthanesulfonique 1000 / 25 / 1 ;
- éluant B : acétonitrile / eau /acide méthanesulfonique 1000 / 25 / 1 ;
- Gradient de 0 à 100 % de B en 10 minutes à 0,81 mL/min ;
- Temps de rétention = 4,51 minutes.

*Spectrométrie de masse (ESI*⁺*) : 529*,*8 [M*+*2H]*²⁺ *; 1058,6 [M*+*H]*⁺ *;*
*Analyse RMN ¹H (600 MHz, DMSO, δ en ppm) : 1,30 (t, 3H) ; 1,38 (qt, 2H) ; 1,59 (qt, 2H) ; 1,69 (s, 12H) ; 1*,*73 (m, 2H) ; 1,96 (qt, 2H) ; 2,12 (t, 2H) ; 2,29 (s, 3H) ; 2*,*45 (dd, 1H) ; 2*,*57 (dd, 1H) ; 2*,*79 (dd, 1H) ; 3,17 (dd, 1H) ; 3*,*41 (q, 2H) ; 3*,*61 (m, 1H) ; 3*,*84 (d, 1H) ; 3*,*97 (t, 2H) ; 4*,*10 (t, 2H) ;* 4,13 *(m, 2H) ; 4,14 (d, 2H) ; 4,17 (d, 1H) ; 6*,*50 (d, 1H) ; 6*,*52 (d, 1H*) *; 6, 65 (dd, 1H*) *; 6*,*71 (d, 1H*) *; 6*,*78 (d, 1H*) *; 6*,*85 (ls, 1H*) *; 6*,*95 (d, 1H*) *; 7, 00 (dd, 1H*) *; 7, 02 (d, 1H*) *; 7*,*08 (d, 1H*) *; 7, 39 (d, 1H*) *; 7*,*69 (m, 2H) ; 7,80 (dl, 1H*) *; 7*,*82 (ls, 2H) ; 8*,*23 (t, 1H*) *; 8*,*34 (t, 1H*) *; 8*,*59 (ls, 1H*) *; 12*,*25 (ls, 1H*) *; 12,91 (ls, 1H)*.

### EXEMPLE 3 : Conjugué Cy™ 5.5 du composé de la Préparation 1

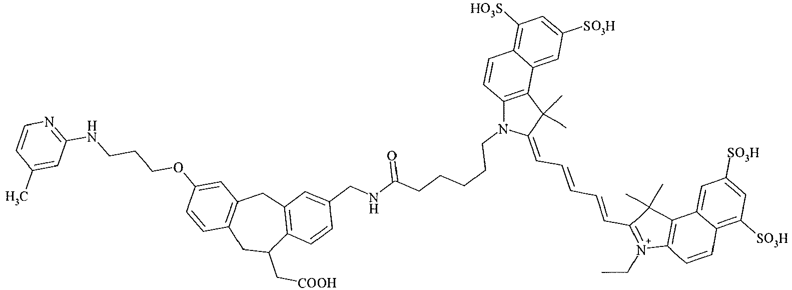

Une solution de composé de la Préparation 1 (9 µmol), de Cyanin5.5 MonoNHS ester (référence PA15602 GE Healthcare) (8,86 µmol) et de 150 µL de triéthylamine dans 2 mL de DMSO est agitée à température ambiante pendant 12 heures. Le brut réactionnel est purifié par chromatographie préparative pour obtenir le composé attendu.

### Conditions de la chromatographie préparative :

- colonne XTerra MS C18 2,5 µm ;
- éluant A : eau / acétonitrile /acide méthanesulfonique 1000 / 25 / 1 ;
- éluant B : acétonitrile / eau /acide méthanesulfonique 1000 / 25 / 1 ;
- Gradient de 0 à 100 % de B en 10 minutes à 0,81 mL/min ;
- Temps de rétention = 4,67 minutes.

*Spectrométrie de masse (ESI*+*) : 672,8 [M*+*2H]*²⁺ *; 683,8 [M*+*H*+*Na]*²⁺ ; *1344,6 [M*+*H]*⁺*.*

*Analyse RMN ¹H (600 MHz, DMSO, δ en ppm) : 1,33 (t, 3H) ; 1,37 (qt, 2H) ; 1,58 (qt, 2H) ; 1,77 (qt, 2H) ; 1,90 (qt, 2H) ; 1,92 (s, 6H) ; 1,95 (2s, 6H) ; 2, 09 (t, 2H) ; 2,27 (s, 3H) ; 2, 43 (dd, 1H*) *; 2,55 (dd, 1H) ; 2*,*75 (dd, 1H) ; 3,12 (dd, 1H*) *; 3, 3 7 (q, 2H) ; 3,81 (d, 1H) ; 3,90 (t, 2H) ; 4,08 (m, 2H) ; 4,13 (d, 1H) ; 4,22 (lt, 2H) ; 4, 26 (ql, 2H) ; 6,34 (d, 2H) ; 6,57 (dd, 1H) ; 6,61 (t, 1H) ; 6, 68 (dd, 1H) ; 6,74 (d, 1H) ; 6,83 (ls, 1H) ; 6,88 (d, 1H) ; 6,97 (dd, 2H) ; 7,04 (m, 1H) ; 7,74 (2d, 2H) ; 7,78 (m, 1H) ; 8,17 (t, 1H) ; 8,20 (d, 1H) ; 8,22 (d, 1H) ; 8,43 (m, 2H) ; 8,44 (s, 1H) ; 8,46 (s, 1H) ; 8,56 (tls, 1H) ; 9,02 (d, 2H) ; 12,90 (ls, 1H).*

### EXEMPLE 4 : Conjugué Cy™ 7 du composé de la Préparation 1

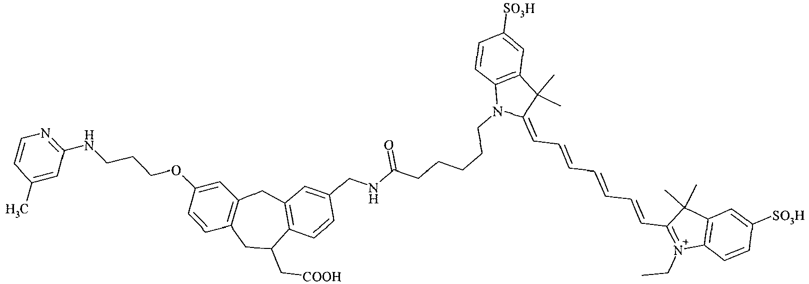

Une solution de composé de la Préparation 1 (13,5 µmol), de Cyanin7 MonoNHS ester (référence PA17102 GE Healthcare) (12,2 µmol) et de 100 µL de triéthylamine dans 2 mL de DMSO est agitée à température ambiante pendant 2 heures. Le brut réactionnel est purifié par chromatographie préparative pour obtenir le composé attendu.

### Conditions de la chromatographie préparative :

- colonne XTerra MS C18 2,5 µm ;
- éluant A : eau / acétonitrile /acide méthanesulfonique 1000 / 25 / 1 ;
- éluant B : acétonitrile / eau /acide méthanesulfonique 1000 / 25 / 1 ;
- Gradient de 0 à 100 % de B en 10 minutes à 0,81 mL/min ;
- Temps de rétention = 4,95 minutes.

*Spectrométrie de masse (ESI*+*) : 555, 8 [M*+*2H]*²⁺ *; 566, 8 [M*+*H*+*Na]*²⁺ *; 574,8 [M*+*H*+*K]*²⁺ *; 1110*,*7 [M*+*H]*⁺*.*

*Analyse RMN ¹H (600 MHz, DMSO, δ en ppm) : 1,26 (t, 3H) ; 1,34 (qt, 2H) ; 1,57 (qt, 2H) ; 1*,*61 (s, 6H) ; 1, 63 (s, 6H) ; 1, 68 (qt, 2H) ; 1*,*95 (qt, 2H) ; 2,11 (t, 2H) ; 2, 30* (*s*, *3H*) *; 2*,*45* (*dd*, *1H*) *; 2*,*57* (*dd*, *1H*) *; 2*,*80* (*dd*, *1H*) *; 3*,*17* (*dd*, *1H*) *; 3*,*41* (*q*, *2H*) *; 3*,*61 (m, 1H*) *; 3*,*85 (d, 1H*) *; 3,97 (t, 2H) ; 4, 03 (lt, 2H) ; 4,12 (lt, 2H) ; 4,14 (d, 2H) ; 4,18 (d, 1H*) *; 6*,*33 (d, 1H*) *; 6,39 (d, 1H*) *; 6,53 (m, 2H) ; 6, 65 (dd, 1H) ; 6,71 (d, 1H) ; 6,79 (d, 1H*) *; 6,84 (ls, 1H*) *; 6,95 (d, 1H*) *; 7,01 (d, 1H*) *; 7,03 (s, 1H*) *; 7,09 (d, 1H*) *; 7,27 (d, 1H*) *; 7,32 (d, 1H*) *; 7,63 (dd, 1H*) *; 7,64 (dd, 1H*) *; 7,72-7,79 (m, 3H) ; 7,80 (d, 1H*) *; 7,85 (t, 1H*)*; 7,90 (t, 1H*) *; 8,21 (t, 1H*) *; 8,30-8,80 (tls, 2H) ; 12,25 (ls, 1H*) *; 12,92 (ls, 1H*).

### EXEMPLE 5 : Conjugué Alexa Fluor® 750 du composé de la Préparation 1

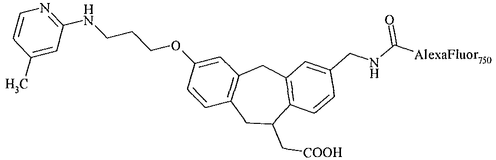

Une solution de composé de la Préparation 1 (4,4 µmol), de l'ester succinimidylique de l'Alexa Fluor® 750 (1,54 µmol) dissous dans 200 (µL de diméthylformamide sec et de triéthylamine séchée sur carbonate de potassium (7,2 µmol) est agitée à température ambiante pendant 1 heure. Le brut réactionnel est purifié par chromatographie préparative pour obtenir le composé attendu.

### Conditions de la chromatographie préparative :

- colonne Prochrom LC50 Symmetry C18 7 µm ;
- éluant A : eau + 0,2 % d'acide trifluoroacétique ;
- éluant B : acétonitrile + 0,2 % d'acide trifluoroacétique ;
- Gradient de 5 à 55 % de B en 30 minutes à 70 mL/min ;
- Temps de rétention = 15,8 minutes.

### EXEMPLE 6 : [¹²⁵I] Acide (7-{[(3-iodobenzoyl)amino]méthyl}-3-{3-[(4-méthyl-2-pyridinyl)amino]propoxy}-10,11-dihydro-5,H-dibenzo[a,d] cyclohepten-10-yl)acétique

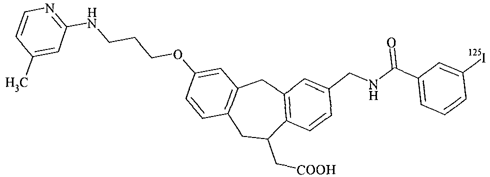

Une solution de composé de la Préparation 1 (24,4 umol), de 1-[(3-iodobenzoyl)oxy]pyrrolidine-2,5-dione (24,4 µmol) dans la triéthylamine séchée sur carbonate de potassium (11 mg) est agitée à température ambiante pendant 1 heure. Le brut réactionnel est purifié sur colonne de silice (éluant : dichlorométhane / méthanol 99 / 1) pour obtenir le composé attendu.

*Spectrométrie de masse (ESI*+*) : 676, 2 [M*+H*]*⁺ *; 1351, 4 [2M*+*H]⁺.*

*Analyse RMN ¹H (400 MHz, DMSO, δ en ppm) : 2,00 (qt, 2H) ; 2, 28 (s, 3H) ; 2,46 (dd, 1H*) *; 2,58 (dd, 1H*) *; 2,83 (dd, 1H*) *; 3,19 (dd, 1H*) *; 3,43 (q, 2H) ; 3, 63 (m, 1H*) *; 3,87 (d, 1H*) *; 4, 02 (t, 2H) ; 4,19 (d, 1H*) *; 4,39 (d, 2H) ; 6, 65 (m, 1H*) *; 6,68 (dd, 1H*) *; 6,76 (m, 1H*) *; 6,81 (d, 1H*) *; 6,98 (d, 1H*) *; 7,11* (*m*, *3H) ; 7,28 (t, 1H*) *; 7,78 (d, 1H*) *; 7,89 (m, 2H) ; 8, 23 (t, 1H*) *; 8,0-8,5 (ls, 1H*) *; 9,04 (t, 1H*) *; 12,19 (ls, 1H*) *; 12,87 (ls, 1H*).

### EXEMPLE 7 : Acide (7-({[(6-hydrazino-3-pyridinyl)carbonyl]amino}méthyl)-3-{3-[(4-méthyl-2-pyridinyl)amino]propoxy}-10,11-dihydro-5H-dibenzo [a,d]cyclohepten-10-yl)acétique

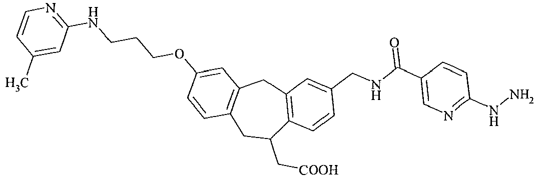

Une solution de composé de la Préparation 1 (1 mmol), de 2-(5-{[(2,5-dioxopyrrolidin-1-yl)oxy]carbonyl}pyridin-2-yl)hydrazinecarboxylate de *tert*-butyle (1 mmol) et de triéthylamine (0,1 mmol) dans 15 mL de diméthylformamide est agitée à température ambiante pendant 16 heures. Le brut réactionnel est trituré avec 15 mL d'éther éthylique et filtré. Le résidu obtenu est traité avec 100 mL d'un mélange acide trifluoroacétique / dichlorométhane 1 / 1 et purifié par chromatographie préparative pour obtenir le composé attendu.

### Conditions_de la chromatographie préparative :

- colonne Prochrom LC50 Symmetry C18 7 µm ;
- éluant A : eau + 0,2 % d'acide trifluoroacétique ;
- éluant B : acétonitrile + 0,2 % d'acide trifluoroacétique ;
- Gradient de 5 à 100 % de B en 30 minutes à 70 mL/min ;
- Temps de rétention = 20,7 minutes.

*Analyse RMN ¹H (400 MHz, DMSO, δ en ppm) : 2.00 (qt, 2H) ; 2*,*30 (s, 3H) ; 2*,*46 (dd, 1H*) *; 2*,*56 (dd, 1H*) *; 2,82 (dd, 1H*) *; 3,19 (dd, 1H*) *; 3*,*45 (m, 2H) ; 3*,*63 (m, 1H*) *; 3*,*87 (d, 1H*) *; 4*,*02* (*t*, *2H) ; 4,19 (d, 1H*) *; 4*,*39* (*d*, *2H) ; 6*,*69 (dd, 1H*) *; 6, 70 (dd, 1H*) *; 6*,*81 (m, 2H) ; 6,89 (d, 1H*) *; 6,98 (d, 1H*) *; 7,11 (m, 3H) ; 7,79 (d, 1H*) *; 8,15 (dd, 1H*) *; 8,49 (ls, 1H) ; 8, 63 (d, 1H*) *; 9, 00 (t, 1H*) *; 9, 73 (ls, 1H*) *; 12, 24 (ls, 1H*).

### EXEMPLE 8: Conjugué Alexa Fluor® 750 du composé de la Préparation 2

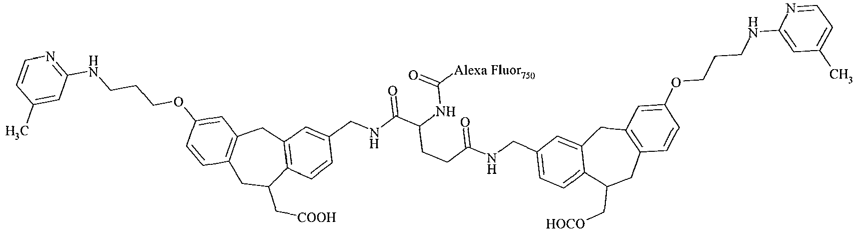

Une solution de composé de la Préparation 2 (1,86 µmol), de l'ester succinimidylique de l'Alexa Fluor® 750 (1,54 µmol) dissous dans 200 µL de diméthylformamide sec et de triéthylamine séchée sur carbonate de potassium (9,4 µmol) est agitée à température ambiante pendant 1 heure. Le brut réactionnel est purifié par chromatographie préparative pour obtenir le composé attendu.

### Conditions de la chromatographie préparative :

- colonne Prochrom LC50 Symmetry C18 7 µm ;
- éluant A : eau + 0,2 % d'acide trifluoroacétique ;
- éluant B : acétonitrile + 0,2 % d'acide trifluoroacétique ;
- Gradient de 5 à 55 % de B en 30 minutes à 70 mL/min ;
- Temps de rétention = 19,7 minutes.

### EXEMPLE 9 : Conjugué entre un Quantum Dot et le composé de la Préparation 1

Une solution de composé de la Préparation 1 (12,8 µmol), de chlorhydrate de 1-éthyl-3-(3-diméthylaminopropyl)carbodiimide (100 mg) dans 10 mL de tampon phosphate salin (pH 7.4) (52 µmol/µL) et du quantum dot T2-MPEviTag™ fonctionnalisé par un groupement carboxyle (E2I61CB20680 ; Evident Technologies) (1,6 nmol) dans 100 µL de DMSO est agitée à température ambiante pendant 2,5 heures.

Le milieu réactionel est concentré à un volume d'environ 400 µL en centrifugeant dans un VIVASPIN 2 (100000 MWCO PES ; Vivascience) à 6000 rpm pendant 5-10 minutes. Le résidu est ensuite lavé 2 fois avec environ 1 mL d'eau Milli-Q puis purifié par chromatographie d'exclusion sur 12 mL de Superdex 200 (diamètre des particules : 24-44 µm) pour obtenir une solution du composé attendu.

### ETUDE PHARMACOLOGIQUE

### EXEMPLE A : Pharmacocinétique plasmatique

Chez des souris Swiss nude femelles (N = 18), on injecte par voie intraveineuse (200 µL), dans la veine caudale, le composé de l'Exemple 3 solubilisé à 10 nmol/mL dans une solution de 0,9 % NaCl. Le sang est collecté à différents moments (5 et 30 minutes puis, 2, 4, 8 et 24 heures ; 3 souris/temps de prélèvement) et, après centrifugation, le plasma surnageant est transféré dans des tubes héparinés. L'échantillon est ensuite dilué dans 50 uL d'un mélange tampon formate d'ammonium 20 mM/acétonitrile/acide formique 50/50/0,1 pour être analysé par UPLC-MS/MS, ESI+.

Après administration intraveineuse chez la souris, la pharmacocinétique du composé de l'Exemple 3 est caractérisée par une distribution rapide suivie d'une élimination lente avec un volume de distribution modéré (1 L/mg), une longue demi-vie (15 heures) et une faible clairance plasmatique (0,8 mL/min/kg) (Figure 1).

### EXEMPLE B : Ciblage de tumeurs greffées en hétérotopique

Dans la patte arrière droite de souris Swiss nude (N = 12), on inocule par voie sous-cutanée de 1 à 2 x 10⁶ cellules tumorales, en formant un groupe de 3 souris pour chaque lignée tumorale (modèle A549 adénocarcinome pulmonaire humain ; modèle H460 carcinome pulmonaire humain; modèle HCT116 carcinome colorectal humain; modèle U87MG glioblastome humain).

Lorsque les tumeurs atteignent un volume compris entre 0,5 et 1 cm, on injecte par voie intraveineuse le composé de l'Exemple 3 (200 µL) solubilisé à 10 nmol/mL dans une solution de tampon phosphate (PBS).

Sous anesthésie gazeuse, on réalise 5 acquisitions à différents moments de l'expérience (2, 4, 6, 8 et 24 heures) à l'aide d'un appareil IVIS Lumina II, Caliper (ex = 680 nm ; em = 720 nm). En parallèle, des prises de sang sont effectuées.

Après euthanasie des animaux par dislocation cervicale, les organes (rein, foie, rate, poumon, tumeur, sang, muscle, coeur, intestin) sont prélevés et une acquisition *ex vivo* est à nouveau effectuée (Lumina II : ex = 680 nm ; em = 720 nm).

### Imagerie de fluorescence du composé de l'Exemple 3 in vivo à 2 nmoles

Comme le montre la Figure 2, quelle que soit la lignée cancéreuse utilisée, les tumeurs ciblées par le composé de l'Exemple 3 sont nettement visualisables 24 heures post injection.

### Cinétique du composé de l'Exemple 3 in vivo

Les cinétiques montrent une fixation tumorale maximale entre 6 et 8 heures post injection puis elles restent stables jusqu'à 24 heures pour chaque lignée de cellules cancéreuses (Figure 3). Toutefois, le meilleur contraste est observé pour les temps plus tardifs lorsque la lente cinétique d'élimination du traceur libre permet d'obtenir un bruit de fond minimal dans les tissus sains.

### Rapport Tissu tumoral / Tissu sain

Selon la lignée tumorale observée A549, H460, HCT116 ou U87MG, les ratios tumeur/tissus sains à t₂₄ₕ sont respectivement de 2,3 ; 1,66; 2 ; et 3,5 (Figure 4). Le contraste augmente progressivement au cours du temps dû à l'élimination lente de la sonde dans le compartiment plasmatique.

### Biodistribution dans les organes et tissus majeurs

L'étude de la biodistribution du composé de l'Exemple 3, dans les principaux organes, 24 heures après injection, démontre une haute spécificité de la sonde pour la tumeur avec une concentration limitée dans les organes les plus volumineux qui est particulièrement favorable à une imagerie *in vivo.* Par exemple, le volume tumoral émet en moyenne 12 fois plus de signal que le tissu musculaire (Figures 5A, 5B et 5C).

L'élimination de ce type de sonde est principalement rénale avec une fixation intrinsèque dans le rein (Figure 6). De plus, le composé de l'Exemple 3 possède une faible fixation rénale (ratio tumeur/rein = 2), propriété particulièrement intéressante pour l'imagerie des foyers abdominaux (Figure 7).

### EXEMPLE C : Ciblage de tumeurs spontanées

Sur le modèle murin K-rasLA1 développant spontanément un carcinome bronchiolo-alvéolaire, on contrôle le développement tumoral par Scanner X synchronisé sur la respiration avant injection du produit à tester. Le composé de l'Exemple 3 (200 µL) solubilisé à 10 nmol/mL dans une solution de tampon phosphate (PBS) est ensuite injecté par voie intraveineuse.

Après euthanasie des animaux, les poumons sont prélevés pour être fixés par injection trachéale de formol neutre. Une acquisition de fluorescence *ex vivo* est effectuée à l'aide d'un appareil IVIS Lumina II, Caliper (ex = 680 nm ; em = 720 nm). Une photographie du poumon excisé est aussi réalisée.

Les résultats montrent que le composé de l'Exemple 3 confirme son fort potentiel de ciblage vis-à-vis des lésions néoplasiques formées *in situ* (Figure 8) et aussi connues pour exprimer les intégrines.

### EXEMPLE D : Interaction avec le développement tumoral

Dans la patte arrière droite de souris Swiss nude (N = 12), on inocule par voie sous-cutanée 3 x 10⁶ cellules tumorales U87MG (glioblastome humain). Lorsque la tumeur atteint un volume de 0,5 cm, on injecte par voie intrapéritonéale la luciférine (2 mg/souris) afin d'effectuer une acquisition en bioluminescence et une quantification à J0.

On divise les animaux en 3 groupes de 4 pour former un groupe contrôle sans injection ; un groupe recevant une injection intraveineuse unique du composé de l'Exemple 3 (2 nmoles dans 200 µL) ; un groupe recevant une injection intraveineuse hebdomadaire pendant 4 semaines du composé de l'Exemple 3 (2 nmoles dans 200 µL). Enfin, l'acquisition en bioluminescence et sa quantification sont réalisées 3 fois par semaine après injection par voie intrapéritonéale de luciférine.

Les résultats montrent que la croissance tumorale est identique pour les trois groupes (Figure 9). Aucun effet anti- ou pro-angiogénique n'est observé selon que l'on réalise une injection unique ou multiple aux concentrations envisagées pour l'imagerie *in vivo* (2 nmoles/souris). Par conséquent, les composés de l'invention, aux doses standards pour l'imagerie, représentent un excellent outil diagnostic fiable sans interaction avec le développement tumoral.

## Revendications

1. Composés de formule (I): 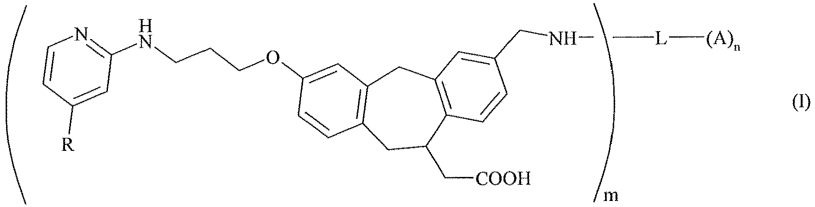 dans laquelle :
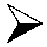 R représente un groupement alkyle(C₁-C₆) linéaire ou ramifié ;
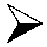 L représente une chaîne hydrocarbonée, linéaire ou ramifiée, saturée ou insaturée, en C₁-C₂₀, étant entendu :
- qu'un ou plusieurs groupements méthylène peuvent également être remplacés par un atome d'oxygène ; un groupement -NH- ; un groupement -CO- ; un groupement hydroxy ; un groupement phényle ; ou un groupement pyridine ; et
- que ladite chaîne hydrocarbonée forme une liaison peptidique -NH-CO- avec l'aminé primaire du ligand de ciblage ;
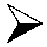 A représente un agent diagnostique correspondant à une entité chimique détectable par médecine nucléaire, par fluorescence, par radioactivité ou par détection optique ;
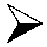 m et n sont chacun indépendamment égaux à 1 ou 2 ;
leurs énantiomères, leurs diastéréoisomères, ainsi que leurs sels d'addition à un acide ou à une base pharmaceutiquement acceptable.

2. Composés de formule (I) selon la revendication 1 **caractérisés en ce que** R représente un groupement méthyle.

3. Composés de formule (I) selon la revendication 1 **caractérisés en ce que** L représente un groupement -CO- ; un groupement (C₁-C₆) alkylcarbonyle ; un groupement phénylcarbonyle ; ou un groupement pyridylcarbonyle.

4. Composés de formule (I) selon la revendication 1 **caractérisés en ce que** L représente le groupement -CO-(CH₂)₅-NH- ; le groupement 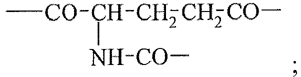 ou le groupement

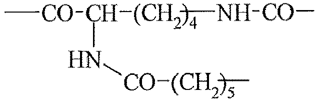

5. Composés de formule (I) selon la revendication 1 **caractérisés en ce que** L représente le groupement *n*-pentylcarbonyle.

6. Composés de formule (I) selon la revendication 1 **caractérisés en ce que** A représente un fluorophore.

7. Composés de formule (I) selon la revendication 1 **caractérisés en ce que** A représente la fluorescéine, l'Alexa Fluor® 750, le Cy™ 3, le Cy™ 5.5 ou le Cy™ 7.

8. Composés de formule (I) selon la revendication 1 **caractérisés en ce que** A représente le Cy™ 5.5.

9. Composés de formule (I) selon la revendication 1 **caractérisés en ce que** m et n sont égaux à 1 ; ou m est égal à 1 et n est égal à 2.

10. Composés de formule (I) selon la revendication 1 qui sont représentés par les formules suivantes :

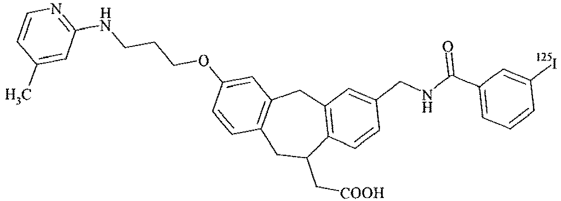

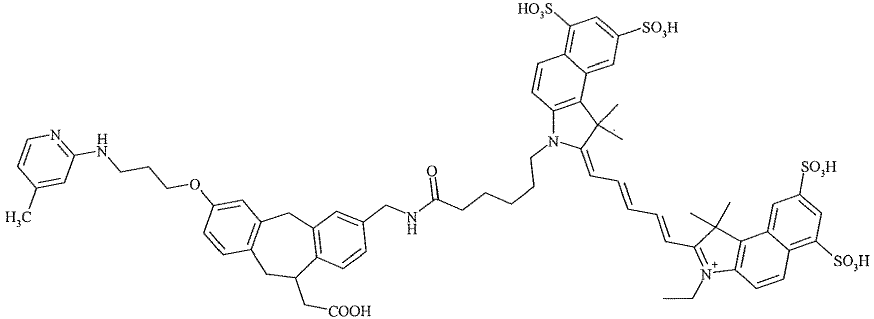

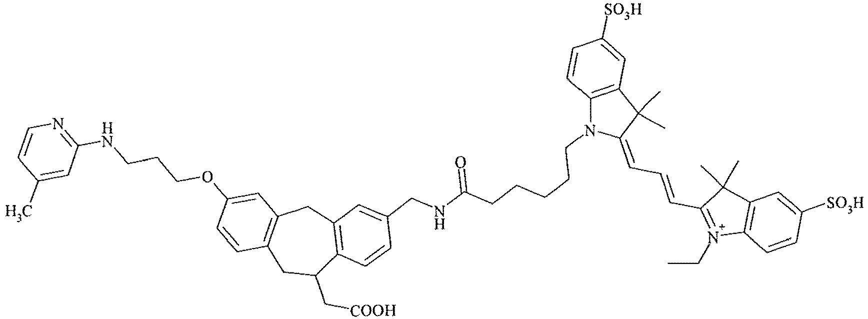

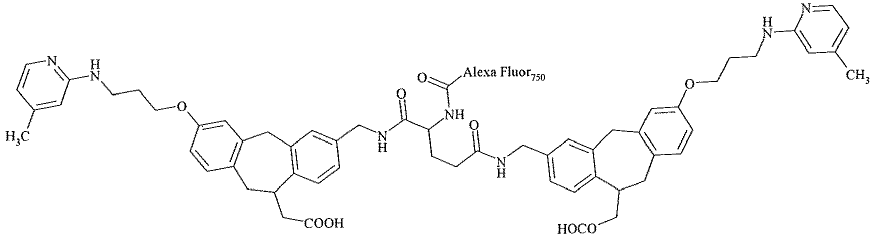

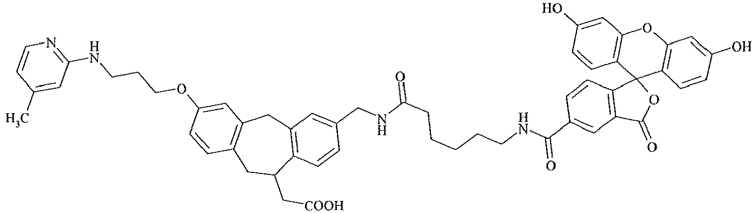

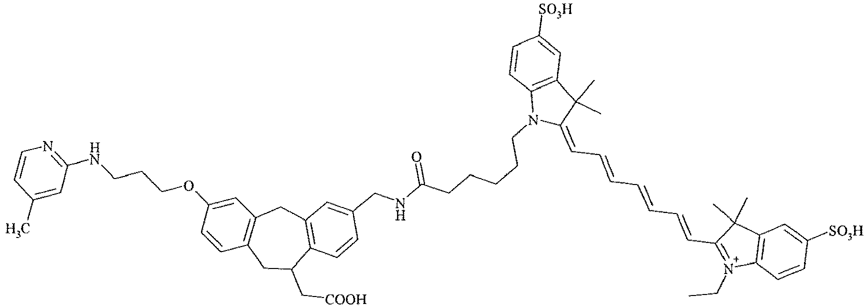

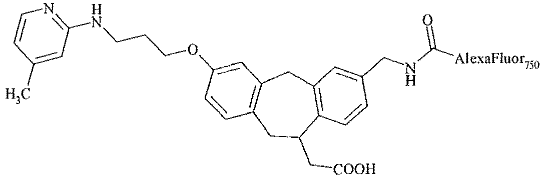

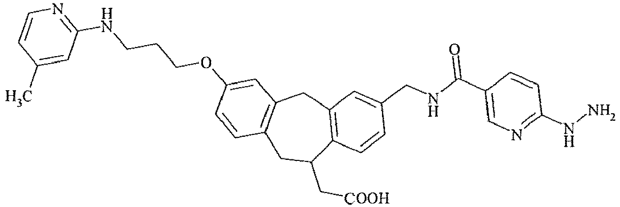

11. Procédé de préparation des composés de formule (I) selon la revendication 1 **caractérisé en ce que** l'on utilise comme produit de départ un composé de formule (II) : 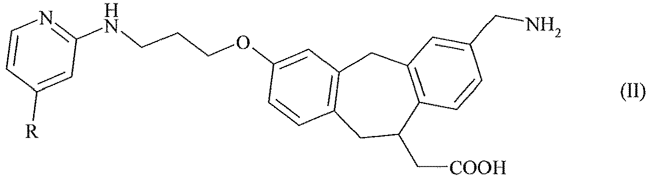 dans laquelle R est tel que défini dans la formule (I),
qui est mis en réaction avec un composé de formule (III) : 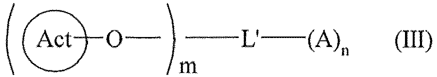 dans laquelle :
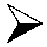 A, m et n sont tels que définis dans la formule (I) ;
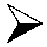 Act représente un activateur de couplage peptique, tel que le *N-*hydroxysuccinimide (NHS), l'éthyl(diméthylaminopropyl)carbodiimide (EDC) ou l'hydroxybenzothiazole (HOBt) ;
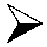 L' représente une chaîne hydrocarbonée, linéaire ou ramifiée, saturée ou insaturée, en C₁-C₂₀, étant entendu :
- qu'un ou plusieurs groupements méthylène peuvent également être remplacés par un atome d'oxygène ; un groupement -NH- ; un groupement -CO- ; un groupement hydroxy ; un groupement phényle ; ou un groupement pyridine ; et
- que la chaîne hydrocarbonée forme une liaison ester avec l'activateur Act ;
pour conduire au composé de formule (I),
composé de formule (I) qui peut être ensuite purifié selon une technique classique de séparation.

12. Ligand de ciblage des récepteurs à intégrines de formule (II) : 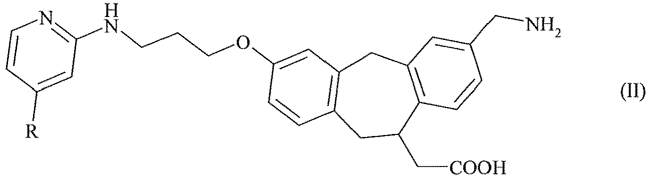 dans laquelle R représente un groupement alkyle (C₁-C₆) linéaire ou ramifié.

13. Ligand de ciblage des récepteurs à intégrines selon la revendication 12 qui est l'acide (7-(aminométhyl)-3-{3-[(4-alkyl-2-pyridinyl)amino]propoxy}-10,11-dihydro-5*H-*dibenzo[*a*,*d*]cyclohepten-10-yl)acétique.

14. Compositions pharmaceutiques contenant comme principe actif un composé selon l'une quelconque des revendications 1 à 10, en combinaison avec un ou plusieurs véhicules inertes, non toxiques et pharmaceutiquement acceptables.

15. Compositions pharmaceutiques selon la revendication 14 utiles à des fins d'imagerie ou de diagnostic in vivo.

16. Un kit pour la préparation d'une composition pharmaceutique selon la revendication 14.

17. Composés de formule (I) selon l'une quelconque des revendications 1 à 10 utiles à des fins d'imagerie ou de diagnostic in vivo.

18. Composés de formule (I) selon l'une quelconque des revendications 1 à 10 utiles pour l'évaluation des effets de l'administration d'un médicament chez l'animal ou chez l'humain atteint d'une pathologie au cours de laquelle une néovascularisation intervient.

## Patentansprüche

1. Verbindungen mit der folgenden Formel (I): 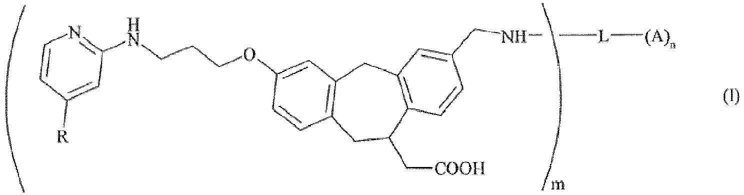 wobei:
- R eine lineare oder verzweigte Alkylgruppe (C₁-C₆) darstellt;
- L eine lineare oder verzweigte, gesättigte oder ungesättigte Kohlenwasserstoffkette mit C₁-C₂₀ darstellt, vorausgesetzt:
-- dass eine oder mehrere Methylengruppen auch durch ein Sauerstoffatom; eine -NH--Gruppe; eine -CO--Gruppe; eine Hydroxygruppe; eine Phenylgruppe; oder eine Pyridingruppe ersetzt werden können; und
-- dass die Kohlenwasserstoffkette eine -NH-CO-- Peptidbindung mit dem primären Amin des Zielliganden bildet;
- A ein Diagnosemittel darstellt, das einer chemischen Einheit entspricht, die mit Hilfe von Nuklearmedizin, durch Fluoreszenz, durch Radioaktivität oder durch optischen Nachweis nachgewiesen werden kann;
- m und n jeweils unabhängig gleich 1 oder 2 sind; ihre Enantiomere, ihre Diastereoisomere, ebenso wie ihre Additionssalze zu einer pharmazeutisch annehmbaren Säure oder Base.

2. Verbindungen mit der Formel (I) nach Anspruch 1, **dadurch gekennzeichnet, dass** R eine Methylgruppe darstellt.

3. Verbindungen mit der Formel (I) nach Anspruch 1, **dadurch gekennzeichnet, dass** L eine -CO--Gruppe; eine (C₁-C₆) Alkylcarbonylgruppe; eine Phenylcarbonylgruppe; oder eine Pyridylcabonylgruppe darstellt.

4. Verbindungen mit der Formel (I) nach Anspruch 1, **dadurch gekennzeichnet, dass** L die Gruppe -CC-(CH₂)₅-NH- ; die Gruppe 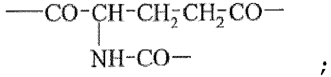 oder die Gruppe 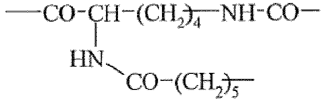 darstellt.

5. Verbindungen mit der Formel (I) nach Anspruch 1, **dadurch gekennzeichnet, dass** L die n-Pentylcarbonylgruppe darstellt.

6. Verbindungen mit der Formel (I) nach Anspruch 1, **dadurch gekennzeichnet, dass** A ein Fluorophor darstellt.

7. Verbindungen mit der Formel (I) nach Anspruch 1, **dadurch gekennzeichnet, dass** A Fluorescein, Alexa Fluor® 750, Cy™ 3, Cy™ 5,5 oder Cy™ 7 darstellt.

8. Verbindungen mit der Formel (I) nach Anspruch 1, **dadurch gekennzeichnet, dass** A Cy™ 5,5 darstellt.

9. Verbindungen mit der Formel (I) nach Anspruch 1, **dadurch gekennzeichnet, dass** m und n gleich 1 sind; oder m gleich 1 und n gleich 2 ist.

10. Verbindungen mit der Formel (I) nach Anspruch 1, die durch die folgenden Formeln dargestellt sind:

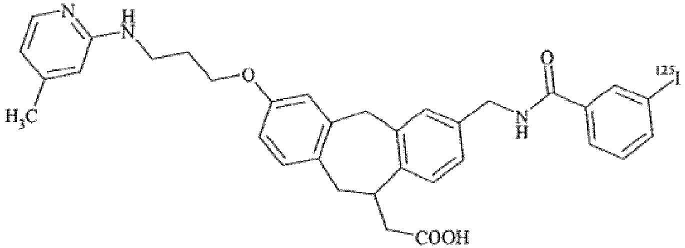

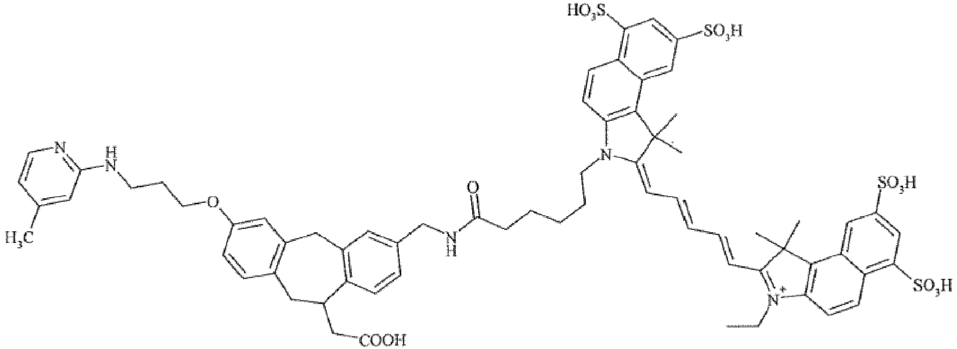

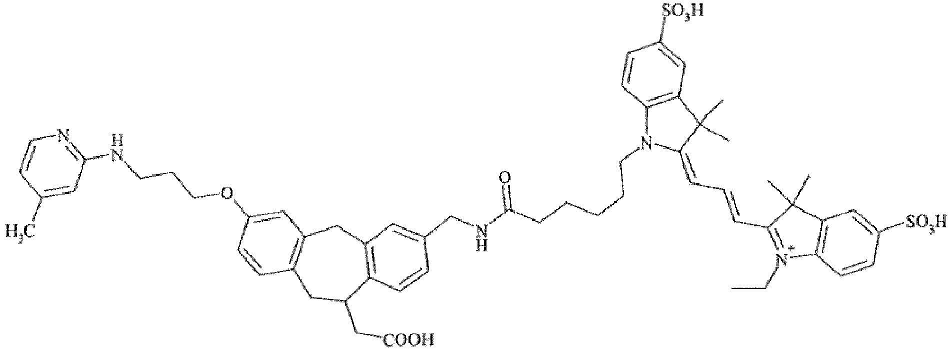

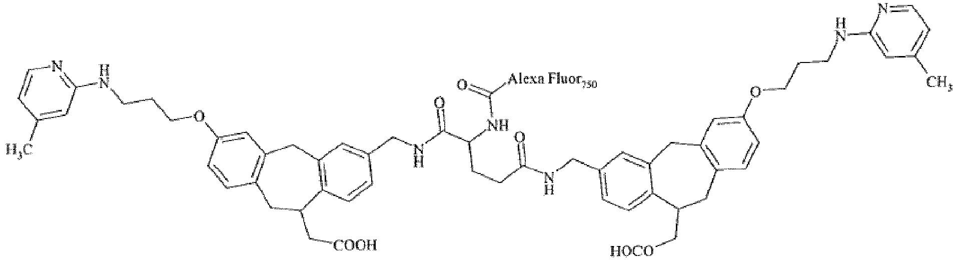

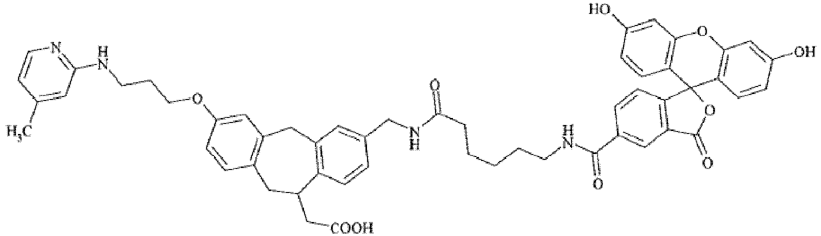

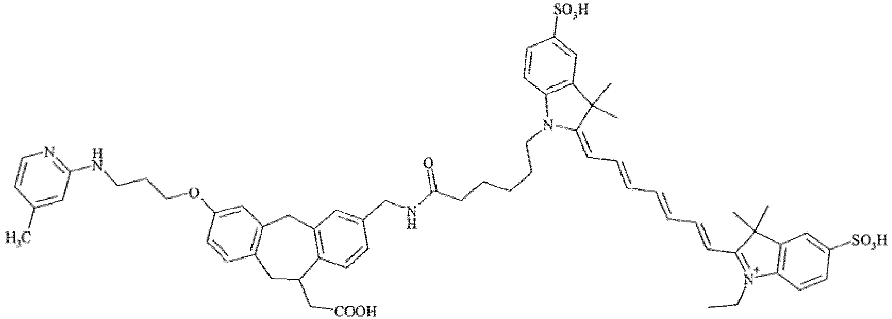

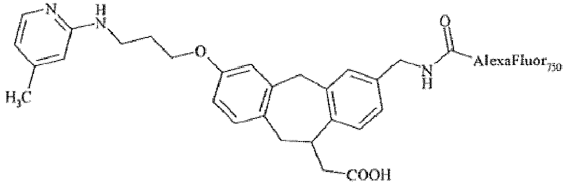

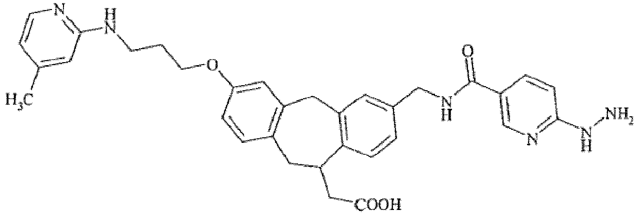

11. Verfahren zur Herstellung der Verbindungen mit der Formel (I) nach Anspruch 1, **dadurch gekennzeichnet, dass** als Ausgangsprodukt eine Verbindung mit der Formel (II) verwendet wird, 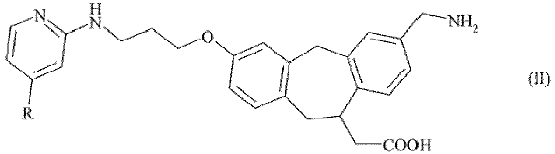 wobei R wie in Formel (I) definiert ist,
die mit einer Verbindung der Formel (III) reagiert wird: 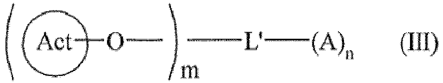 wobei:
- A, m und n wie in Formel (I) definiert sind;
- Act einen Aktivator der Peptidkupplung darstellt, wie z.B. N-Hydroxysuccinimid (NHS), Ethyl(dimethylaminopropyl)carbodiimid (EDC) oder Hydroxybenzothiazol (HOBt);
- L' eine lineare oder verzweigte, gesättigte oder ungesättigte Kohlenwasserstoffkette mit C₁-C₂₀ darstellt, vorausgesetzt:
-- dass eine oder mehrere Methylengruppen auch durch ein Sauerstoffatom; eine -NH--Gruppe; eine -CO--Gruppe; eine Hydroxygruppe; eine Phenylgruppe; oder eine Pyridingruppe ersetzt werden können; und
-- dass die Kohlenwasserstoffkette eine Esterbindung mit dem Aktivator Act bildet;
um zur Verbindung mit der Formel (I) zu gelangen, Verbindung mit der Formel (I), die dann gemäß einer klassischen Trenntechnik gereinigt werden kann.

12. Zielligand der Rezeptoren mit Integrinen mit der Formel (II) : 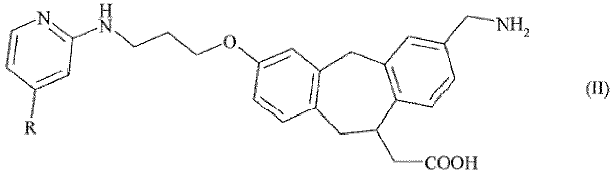 wobei R eine lineare oder verzweigte Alkylgruppe (C₁-C₆) darstellt.

13. Zielligand der Rezeptoren mit Integrinen nach Anspruch 12, wobei es sich um die (7-(Aminomethyl)-3-{3-[(4-alkyl-2-pyridinyl)amino]propoxy}-10,11-dihydro-5H-dibenzo[a,d]cyclohepten-10-yl)essigsäure handelt.

14. Pharmazeutische Zusammensetzungen, umfassend als Wirkstoff eine Verbindung nach einem der Ansprüche 1 bis 10 in Kombination mit einem oder mit mehreren inerten, nicht toxischen und pharmazeutisch annehmbaren Vehikeln.

15. Pharmazeutische Zusammensetzungen nach Anspruch 14, die für Bildgebungen oder eine Diagnose *in vivo* nützlich sind.

16. Kit zur Herstellung einer pharmazeutischen Zusammensetzung nach Anspruch 14.

17. Verbindungen mit der Formel (I) nach einem der Ansprüche 1 bis 10, die für Bildgebungen oder eine Diagnose *in vivo* nützlich sind.

18. Verbindungen mit der Formel (I) nach einem der Ansprüche 1 bis 10, die für die Bewertung der Wirkungen der Verabreichung eines Medikaments beim Tier oder beim Menschen nützlich sind, die an einer Pathologie leiden, im Verlauf derer eine Neovaskularisation auftritt

## Claims

1. Compounds of formula (I): 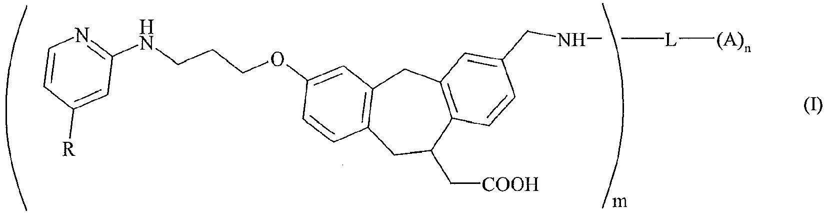 wherein:
- R is a linear or branched (C₁-C₆) alkyl group;
- L is a C₁-C₂₀ saturated or unsaturated, linear or branched hydrocarbon chain, given that:
- one or more methylene groups can also be replaced by an oxygen atom; a -NH- group; a
- CO- group; a hydroxy group; a phenyl group; or a pyridine group; and
- said hydrocarbon chain forms a -NH-CO- peptide bond with the primary amine of the targeting ligand;
- A is a diagnostic agent corresponding to a chemical entity detectable by nuclear medicine, by fluorescence, by radioactivity or by optical detection;
- m and n are each independently equal to 1 or 2;
enantiomers thereof, diastereoisomers thereof, as well as pharmaceutically acceptable acid or base addition salts thereof.

2. Compounds of formula (I) according to claim 1, **characterized in that** R is a methyl group.

3. Compounds of formula (I) according to claim 1, **characterized in that** L is a -CO- group; a (C₁-C₆) alkylcarbonyl group; a phenylcarbonyl group; or a pyridylcarbonyl group.

4. Compounds of formula (I) according to claim 1, **characterized in that** L is the -CO-(CH₂)₅-NH- group; the 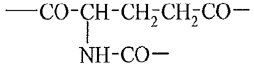 group; or the 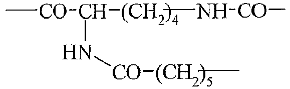 group.

5. Compounds of formula (I) according to claim 1, **characterized in that** L is the *n*-pentylcarbonyl group.

6. Compounds of formula (I) according to claim 1, **characterized in that** A is a fluorophore.

7. Compounds of formula (I) according to claim 1, **characterized in that** A is fluorescein, Alexa Fluor^{®} 750, Cy™ 3, Cy™ 5.5 or Cy™ 7.

8. Compounds of formula (I) according to claim 1, **characterized in that** A is Cy™ 5.5.

9. Compounds of formula (I) according to claim 1, **characterized in that in that** m and n are equal to 1; or m is equal to 1 and n is equal to 2.

10. Compounds of formula (I) according to claim 1, represented by the following formulas:

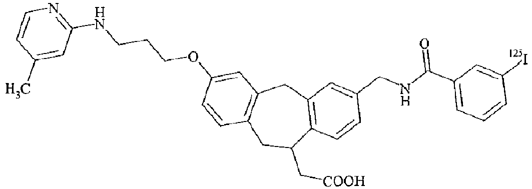

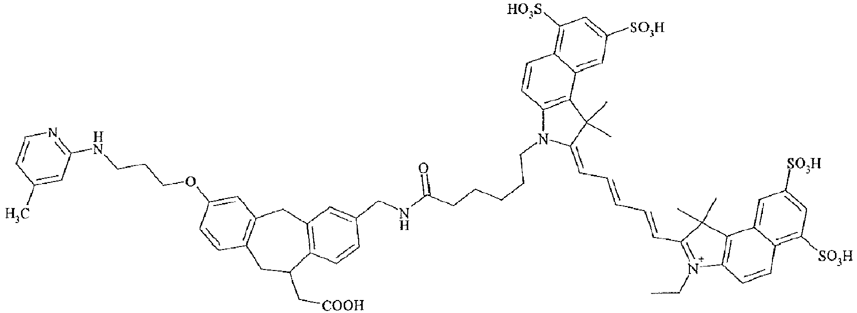

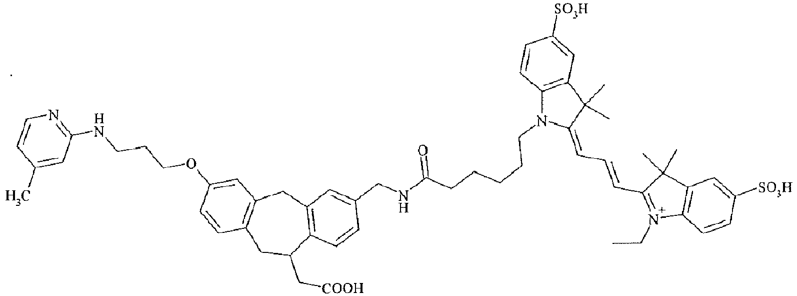

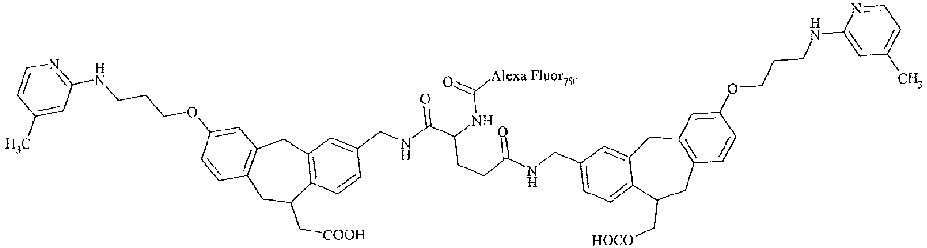

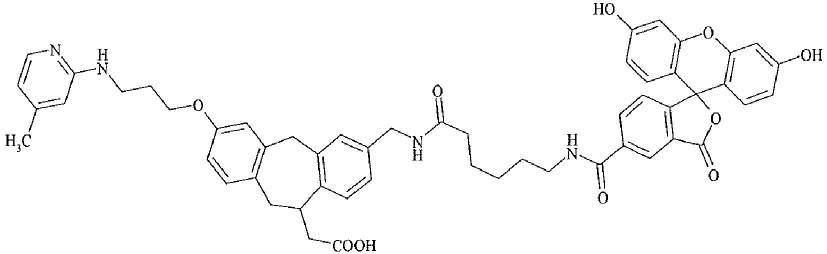

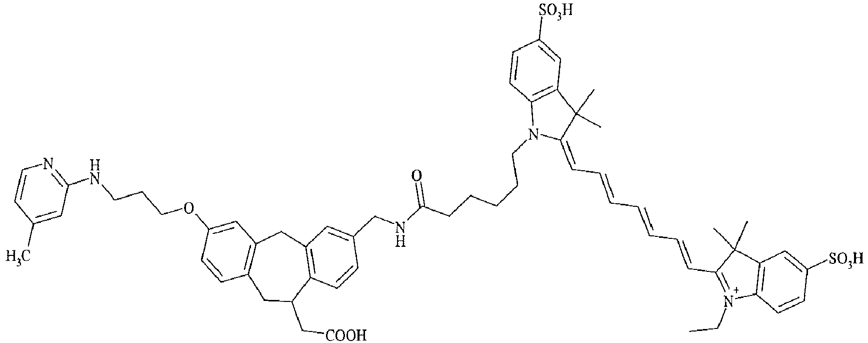

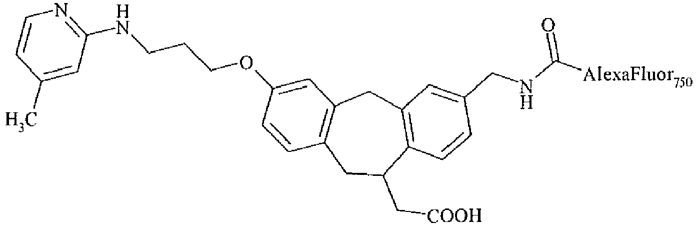

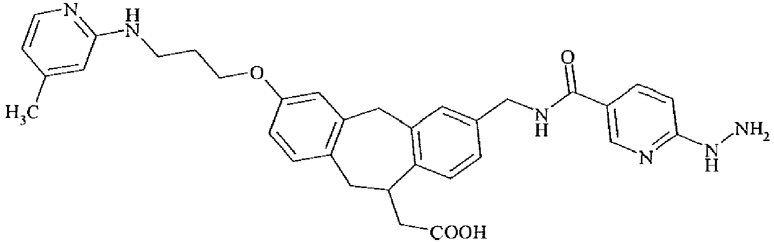

11. A method for the preparation of compounds of formula (I) according to claim 1, **characterized in that** the starting product used is a compound of formula (II): 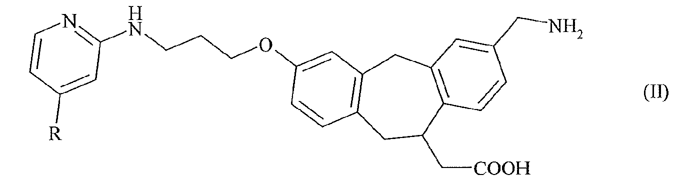 wherein R is as defined in formula (I),
which is reacted with a compound of formula (III): 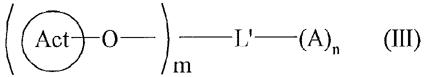 wherein:
- A, m and n are as defined in formula (I);
- Act is a peptide coupling activator, such as N-hydroxysuccinimide (NHS), ethyl(dimethylaminopropyl)carbodiimide (EDC) or hydroxybenzothiazole (HOBt);
- L' is a C₁-C₂₀ saturated or unsaturated, linear or branched hydrocarbon chain, given that:
- one or more methylene groups can also be replaced by an oxygen atom; a -NH- group; a -CO- group; a hydroxy group; a phenyl group; or a pyridine group; and
- the hydrocarbon chain forms an ester bond with the activator Act;
to lead to the compound of formula (I),
compound of formula (I) which can then be purified according to a standard separation technique.

12. An integrin receptor targeting ligand of formula (II) : 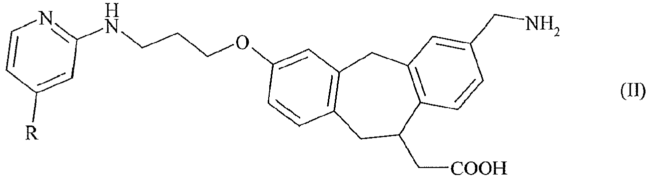 wherein R is a linear or branched (C₁-C₆) alkyl group.

13. The integrin receptor targeting ligand according to claim 12, which is (7-(aminomethyl)-3-{3-[(4-alkyl-2-pyridinyl)amino]propoxy}-10,11-dihydro-5*H-*dibenzo[*a*,*d*]cyclohepten-10-yl)acetic acid.

14. Pharmaceutical compositions containing as active ingredient a compound according to any one of claims 1 to 10, in combination with one or more inert, nontoxic and pharmaceutically acceptable carriers.

15. Pharmaceutical compositions according to claim 14, useful for purposes of imaging or *in vivo* diagnosis.

16. A kit for the preparation of a pharmaceutical composition according to claim 14.

17. Compounds of formula (I) according to any one of claims 1 to 10, useful for purposes of imaging or *in vivo* diagnosis.

18. Compounds of formula (I) according to any one of claims 1 to 10, useful for the evaluation of the effects of the administration of a drug to an animal or human suffering from a pathology during which neovascularization occurs.
